(19) Europäisches Patentamt
European Patent Office
Office européen des brevets

(11) EP 4 292 599 A1

(12) **EUROPEAN PATENT APPLICATION**
published in accordance with Art. 153(4) EPC

(43) Date of publication:
**20.12.2023 Bulletin 2023/51**

(21) Application number: **21930411.0**

(22) Date of filing: **21.12.2021**

(51) International Patent Classification (IPC):
***A61K 35/28*** *(2015.01)* ***A61K 35/32*** *(2015.01)*
***A61P 19/02*** *(2006.01)* ***C12Q 1/06*** *(2006.01)*
***G01N 33/48*** *(2006.01)*

(52) Cooperative Patent Classification (CPC):
**A61K 35/28; A61K 35/32; A61P 19/02; C12Q 1/06; G01N 33/48**

(86) International application number:
**PCT/JP2021/047431**

(87) International publication number:
**WO 2022/190566 (15.09.2022 Gazette 2022/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.03.2021 JP 2021040648**

(71) Applicants:
- **FUJIFILM Corporation**
  **Tokyo 106-8620 (JP)**
- **National University Corporation Tokyo Medical and Dental University**
  **Tokyo 113-8510 (JP)**

(72) Inventors:
- **NAKAMURA, Kentaro**
  **Ashigarakami-gun, Kanagawa 258-8577 (JP)**
- **TOMINAGA, Shunsuke**
  **Ashigarakami-gun, Kanagawa 258-8577 (JP)**
- **SEKIYA, Ichiro**
  **Tokyo 113-8510 (JP)**
- **MIZUNO, Mitsuru**
  **Tokyo 113-8510 (JP)**
- **KATANO, Hisako**
  **Tokyo 113-8510 (JP)**
- **OZEKI, Nobutake**
  **Tokyo 113-8510 (JP)**

(74) Representative: **Hoffmann Eitle**
**Patent- und Rechtsanwälte PartmbB**
**Arabellastraße 30**
**81925 München (DE)**

(54) **METHOD FOR PRODUCING THERAPEUTIC AGENT FOR ARTHROPATHY, AND THERAPEUTIC AGENT FOR ARTHROPATHY**

(57) There are provided a production method for a therapeutic agent for arthrosis, in which a population including a plurality of mesenchymal stem cells being cultured is imaged to acquire a cell image, the cell image is analyzed to derive density information on the mesenchymal stem cells in the cell image, colonies, which are formed by the mesenchymal stem cells in the population, are detected based on the density information, and the population is sorted based on at least one of an average colony size or a colony forming unit, which is derived from the detected colonies, and provided a therapeutic agent for arthrosis, which is produced by the production method for a therapeutic agent for arthrosis.

FIG. 1

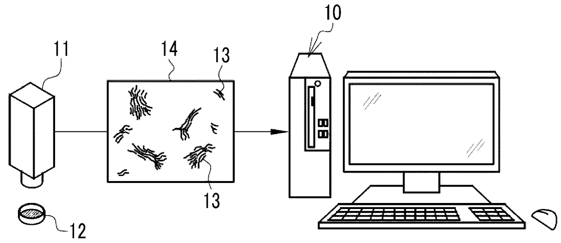


EP 4 292 599 A1

## Description

BACKGROUND OF THE INVENTION

1. Field of the Invention

The present disclosure relates to a production method for a therapeutic agent for arthrosis, and a therapeutic agent for arthrosis.

2. Description of the Related Art

In the field of orthopedics, arthrosis such as articular cartilage injury and meniscus injury are frequently seen as subjects of daily medical practice and are widely recognized as diseases from which a large number of patients suffer.

As an example of surgical medical treatment for cartilage injury or meniscus injury, a surgical treatment of removing debris in the injury part is carried out.

Although the above-described treatment suppresses the progression of the medical condition and promotes the self-renewal of the tissue, the self-renewal of the tissue has not been sufficient.

On the other hand, cell therapy has become popular due to recent advances in regenerative medicine technology. In particular, the mesenchymal stem cell (MSC) is expected as a cell source useful for a cell therapy. The mesenchymal stem cells can be collected from various biological tissues and have been reported to be capable of being isolated from the bone marrow tissue (Prockop, D.J., 1997, Science. 276:71-4), the adipose tissue (Zuk, P.A. et al., 2002, Mol Biol Cell. 13:4279-95), the muscle tissue (Cao et al., 2003, Nat Cell Biol. 5:640-6), the synovial tissue (De Bari, C. et al., 2001, Arthritis Rheum. 44:1928-42), the periosteal tissue (Fukumoto, T. et al., 2003, Osteoarthritis Cartilage. 11:55-64), and the like.

In addition, it has been reported that synovium-derived mesenchymal stem cells have an excellent proliferative ability and cartilage forming ability as compared with mesenchymal stem cells derived from various mesenchymal tissues such as bone marrow (Sakaguchi, et al., 2005, Arthritis Rhum. 52:2521-9).

The mesenchymal stem cells are proliferated until a desired number thereof is obtained by culturing and then transplanted to an injury part as a therapeutic agent for arthrosis. However, the information on whether or not the mesenchymal stem cells reach the desired number by the day when they are transplanted is important, and thus it is strongly desired that the above information is obtained in the middle of the culture period.

In response to the above requirement, for example, JP2019-4794A proposes a proliferation prediction method including a calculation step of calculating an indicator for predicting a future proliferation state of mesenchymal stem cells based on a culturing state of the mesenchymal stem cells at a first time point, and a prediction step of predicting a proliferation state of the mesenchymal stem cells at a second time point after the first time point based on the indicator and a discriminant expression.

SUMMARY OF THE INVENTION

A colony formed by mesenchymal stem cells are different from colonies of other cells such as epithelial-like cells and lymphoblast-like cells, and the boundary is unclear.

In JP2019-4794A, it is determined that a colony is formed in a case where four or more cells are confirmed when the number of cells present at a certain distance from a certain cell is counted.

The inventors of the present invention novelly found that in the colony determination method adopted in the proliferation prediction method of JP2019-4794A, in a case where proliferated mesenchymal stem cells are densely present, it is determined that these cells belong to one colony, and thus there is a risk that the number of cells belonging to one colony becomes too large and an accurate proliferation prediction is difficult.

The present disclosure has been made in consideration of the above circumstances, and an object to be achieved by the present disclosure is to provide a production method for a therapeutic agent for arthrosis, which makes it possible to carry out a proliferation prediction of mesenchymal stem cells with excellent accuracy in the middle of culturing and makes it possible to produce a therapeutic agent for arthritis, suitable for the medical treatment of arthritis, and to provide a therapeutic agent for arthrosis, which is produced by the production method for a therapeutic agent for arthrosis.

<1> A production method for a therapeutic agent for arthrosis, comprising:

imaging a population including a plurality of mesenchymal stem cells being cultured, to acquire a cell image;
analyzing the cell image to derive density information on the mesenchymal stem cells in the cell image;

detecting colonies, which are formed by the mesenchymal stem cells in the population, based on the density information; and

sorting the population based on at least one of an average colony size or a colony forming unit, which is derived from the detected colonies.

<2> The production method for a therapeutic agent for arthrosis according to <1>, in which the density information is an estimated density distribution of the mesenchymal stem cells in the cell image, which is derived from kernel density estimation.

<3> The production method for a therapeutic agent for arthrosis according to <2>, in which the detection of the colonies is carried out by carrying out a mean-shift with reference to the estimated density distribution and detecting, as the colony, a collection of the mesenchymal stem cells, which converges to the same maximal point in the estimated density distribution.

<4> The production method for a therapeutic agent for arthrosis according to any one of <1> to <3>, in which the detection of the colonies is carried out on a 4th day to a 6th day from a culture start date, and the sorting of the population is carried out by sorting a population that satisfies at least one of the following condition a1) or b1),

a1) the average colony size of the mesenchymal stem cells included in the population at any time point of the 4th day to the 6th day from the culture start date is 0.740 mm or more, or

b1) the colony forming unit of the mesenchymal stem cells included in the population at any time point of the 4th day to the 6th day from the culture start date is 0.250 units/$mm^2$ or more.

<5> The production method for a therapeutic agent for arthrosis according to any one of <1> to <4>, in which the mesenchymal stem cells are obtained by treating a biological tissue with an enzyme.

<6> The production method for a therapeutic agent for arthrosis according to <5>, in which in the treatment of the biological tissue with an enzyme, an enzyme mixture containing at least one kind of collagenase and at least one kind of neutral protease is used.

<7> The production method for a therapeutic agent for arthrosis according to <5> or <6>, in which in the treatment of the biological tissue with an enzyme, a ratio of a using amount of the biological tissue to a using amount of the enzyme is 10 to 1,000 on a mass basis.

<8> The production method for a therapeutic agent for arthrosis according to any one of <5> to <7>, in which the mesenchymal stem cells are obtained by washing a tissue digestion solution which is obtained by the treatment of the biological tissue with an enzyme until a residual enzyme concentration in a supernatant is 0.5 ng/mL or less.

<9> The production method for a therapeutic agent for arthrosis according to any one of <1> to <8>, in which the mesenchymal stem cells are a synovium-derived cell.

<10> The production method for a therapeutic agent for arthrosis according to any one of <1> to <9>, in which the mesenchymal stem cells are human-derived cells.

<11> A therapeutic agent for arthritis, which is produced by the production method for a therapeutic agent for arthrosis according to any one of <1> to <10>.

According to the present disclosure, it is possible to provide a production method for a therapeutic agent for arthrosis, which makes it possible to carry out a proliferation prediction of mesenchymal stem cells with excellent accuracy in the middle of culturing and makes it possible to produce a therapeutic agent for arthritis, suitable for the medical treatment of arthritis, and to provide a therapeutic agent for arthrosis, which is produced by the production method for a therapeutic agent for arthrosis.

BRIEF DESCRIPTION OF THE DRAWINGS

Fig. 1 is a view illustrating a cell image analysis device and the like.

Fig. 2 is a block diagram illustrating a computer that constitutes the cell image analysis device.

Fig. 3 is a block diagram illustrating a processing unit of a CPU of the cell image analysis device.

Fig. 4 is a view illustrating details of a density information derivation unit.

Fig. 5 is a graph showing an outline of kernel density estimation.

Fig. 6 is a graph showing an outline of a mean-shift.

Fig. 7 is a graph showing an outline of the mean-shift.

Fig. 8 is a view illustrating details of a detection result.

Fig. 9 is a view illustrating details of evaluation information.

Fig. 10 is a view illustrating a size of a colony.
Fig. 11 is a view illustrating a cell image display screen.
Fig. 12 is a view illustrating an analysis result display screen.
Fig. 13 is a view illustrating a state in which a specific region is subjected to kernel density estimation with three kinds of band widths to derive three kinds of estimated density distributions.
Fig. 14 is a view illustrating a state in which a specific region is subjected to a mean-shift with reference to each of the three kinds of estimated density distributions to output detection results of three kinds of colonies, which respectively match with the three kinds of estimated density distributions.
Fig. 15 is a view illustrating a designation screen.
Fig. 16 is a view illustrating a state in which a designation of a band width is received by an instruction receiving unit.
Fig. 17 is a flowchart illustrating a processing procedure of the cell image analysis device.
Fig. 18 is the flowchart illustrating the processing procedure of the cell image analysis device.
Fig. 19 is a view illustrating another example of the evaluation information.
Fig. 20 is a view illustrating still another example of the evaluation information.
Fig. 21 is a view illustrating an aspect in which a warning is displayed in a case where a change in an amount of an average colony size per one day of culturing is less than a threshold value.
Fig. 22 is a flowchart illustrating another example of a designation method for a designated band width.
Fig. 23 is a view illustrating a second embodiment in which derivation of density information and detection of colonies are executed only in a case where an area ratio of fibroblast-like cells in a cell image is within a set range.
Fig. 24 is a view illustrating another example according to the second embodiment.
Fig. 25 is a view illustrating still another example according to the second embodiment.
Fig. 26 is a view summarizing the aspects shown in Fig. 23 to Fig. 25 in a graph in which the vertical axis indicates the area ratio and the horizontal axis indicates the number of days of culture.
Fig. 27 is a view illustrating a cell image display screen in a case where the area ratio is outside the set range.
Fig. 28 is a view illustrating a third embodiment in which the number of fibroblast-like cells in a preset search frame is derived as the density information.
Fig. 29 is a view illustrating a state in which search frames are sequentially scanned in a plurality of regions in a cell image.
Fig. 30 is a view illustrating a case where the number of fibroblast-like cells in the search frame satisfies the colony condition.
Fig. 31 is a view illustrating a fourth embodiment in which image analysis is carried out using a machine learning model.
Fig. 32 is a view illustrating an outline of processing in a learning phase of the machine learning model.

DESCRIPTION OF THE PREFERRED EMBODIMENTS

Hereinafter, embodiments for executing the present disclosure will be described in detail. However, the present disclosure is not limited to the following embodiments. In the following embodiments, constitutional elements of the embodiments are not essential unless otherwise specified. The same applies to numerical values and ranges of the numerical values, which do not limit the present disclosure.
In the present disclosure, the "mesenchymal stem cell" is a somatic stem cell derived from a mesodermal tissue (mesenchyme).
The mesenchymal stem cells are known to be present in bone marrow, synovium, periosteum, adipose tissue, and muscle tissue, and they are known to be capable of differentiating into osteoblasts, cartilage cells, adipose cells, and muscle cells. In relation to the differentiation of mesenchymal stem cells into cartilage cells, it is known that the addition of BMP or TGF-β to the culture solution promotes the differentiation of undifferentiated mesenchymal stem cells into cartilage cells, and the cartilage tissue can be regenerated under in vitro conditions.
The mesenchymal stem cell can be confirmed by detecting a molecule characteristic of the mesenchymal stem cell (an enzyme, a receptor, a low-molecular-weight compound, or the like).
Examples of the molecule characteristic of mesenchymal stem cells include, which are not limited to, CD73, CD90, CD105, and CD166, which are cell surface markers (positive markers).
In addition, examples of the cell surface marker (a negative marker) that is not expressed in the mesenchymal stem cell include Clusters of Differentiation (CD) 19, CD34, CD45, Human Leukocyte Antigen-D-Related (HLA-DR), CD11b, and CD14, which are not limited thereto.
The above-described positive markers and negative markers can be used to confirm that a cell is the mesenchymal stem cell.
Although an immunological method can be used for the detection of these markers, the detection may be carried out by quantifying the mRNA amount of each molecule.
In the present disclosure, the "population" means a system in which mesenchymal stem cells are cultured, and the mesenchymal stem cells form colonies in the population.

In the present disclosure, the "average colony size" means an average value of sizes of colonies formed by mesenchymal stem cells.

In a case where the colony size is equal to or smaller than a certain value, there is a high possibility that isolated cells or dust in the culture medium is detected. Therefore, in the present disclosure, the measurement of the average colony size is carried out with respect to colonies having a colony size of more than 0.54 mm.

In the present disclosure, the "colony forming unit" is also referred to as CFU and means the number of colonies per 1 $mm^2$ of a cell image. It is noted that the cell image will be described later.

In the present disclosure, the "arthrosis" is a disease associated with injury, damage, or inflammation of a joint.

More specifically, examples of arthrosis include meniscus injury, traumatic cartilage injury, osteochondritis dissecans, aseptic osteonecrosis, osteoarthritis (for example, knee osteoarthritis, elbow osteoarthritis, and shoulder osteoarthritis), rheumatoid arthritis (for example, chronic rheumatoid arthritis), gout, reactive arthritis, psoriatic arthritis, juvenile arthritis, inflammatory arthritis, and articular cartilage defect, which are not limited to these diseases.

In the present disclosure, a numerical range described using "to" includes numerical values before and after "to" as a minimum value and a maximum value, respectively.

In the numerical ranges described stepwise in the present disclosure, the upper limit value or the lower limit value described in one numerical range may be replaced with the upper limit value or the lower limit value of the numerical range described stepwise in other stages. Further, in the numerical ranges described in the present disclosure, the upper limit value or the lower limit value of the numerical range may be replaced with the value shown in Examples.

(Production method for therapeutic agent for arthrosis)

In a production method for a therapeutic agent for arthrosis according to the present disclosure, the following procedure is carried out:

a population including a plurality of mesenchymal stem cells being cultured is imaged to acquire a cell image;
the cell image is analyzed to derive density information on the mesenchymal stem cells in the cell image; colonies, which are formed by the mesenchymal stem cells in the population, are detected based on the density information; and the population is sorted based on at least one of an average colony size or a colony forming unit, which is derived from the detected colonies.

According to the production method for a therapeutic agent for arthrosis according to the present disclosure, it is possible to provide a production method for a therapeutic agent for arthrosis, which makes it possible to carry out a proliferation prediction of mesenchymal stem cells with excellent accuracy in the middle of culturing and makes it possible to produce a therapeutic agent for arthritis, suitable for the medical treatment of arthritis.

The reason why the above effect is obtained is presumed as follows, which is not limited thereto.

As described above, in the proliferation prediction for mesenchymal stem cells in the related art which has been proposed in JP2019-4794A or the like, there is a case where it is difficult to carry out the detection of colonies with excellent accuracy due to the unclearness of the boundary of the colony formed by the mesenchymal stem cells, and thus there has been room for improvement in the accuracy of proliferation prediction.

In the production method for a therapeutic agent for arthrosis according to the present disclosure, density information on mesenchymal stem cells are derived, and colonies which are formed by the mesenchymal stem cells in the population are detected based on the density information, which makes it possible to detect colonies of mesenchymal stem cells with excellent accuracy.

It is presumed that a therapeutic agent for arthrosis suitable for the medical treatment of arthrosis can be produced since from the information on the colonies detected with excellent accuracy, the average colony size and the colony forming unit are derived, and based on these, a population of the mesenchymal stem cells, which is excellent in the degree of proliferation, the proliferation rate, and the like, is sorted.

(Culturing of mesenchymal stem cell)

For culturing of the mesenchymal stem cells, a general culture medium that is used for culturing animal cells can be used.

Examples of the culture medium include α-MEM, a Dulbecco Modified Eagle Medium (DMEM), a mixed culture medium of DMEM and F12 (DMEM:F12 = 1:1), a Roswell Park Memorial Institute (RPMI) medium (GIBCO (registered trade name) RPMI 1640 medium, or the like), and a mixed culture medium obtained by mixing the mixed culture medium of DMEM and F12 with the RPMI medium (mixed culture medium of DMEM and F12:RPMI medium = 1:1), which is not limited thereto.

It is noted that the ratio in parentheses is a ratio on the mass basis.

The culture medium may be a culture medium containing serum or may be a culture medium containing no serum.

In a case of producing mesenchymal stem cells from the autologous tissue for the intended purpose of transplantation to a living body, the culture medium may contain allogeneic serum.

That is, in a case of producing a mesenchymal stem cell from a human tissue for the intended purpose of transplantation to a human, a culture medium containing a human serum may be used.

In a case where serum is used, the serum may be autologous serum or may be allogeneic serum; however, it is preferably autologous serum. In a case where serum is used, the amount of serum to be added to the culture medium is preferably 20% by volume or less and more preferably 10% by volume or less.

The cell culturing conditions are not particularly limited, and general cell culturing conditions can be employed.

For example, the temperature of the cell culture can be 30°C to 40°C.

In addition, the $CO_2$ concentration of the cell culture can be 3% to 7%.

As an example, conditions of a temperature of 37°C and a $CO_2$ concentration of 5% can be adopted, which are not limited thereto.

It is preferable that the culture medium is not exchanged during the culture period.

In addition, in a case where the culture period is 10 days or more, it is preferable that the mesenchymal stem cells are cultured without being co-cultured with cells other than the mesenchymal stem cells.

The differentiation of mesenchymal stem cells into cartilage cells progresses as the culture period increases.

Therefore, it is known that the in situ cartilage forming ability of mesenchymal stem cells decreases when the culture period exceeds a specific length.

It is preferable to adjust the culture period in order to proliferate mesenchymal stem cells in an undifferentiated state and in a state of having a good in situ cartilage forming ability.

In addition, in order to regenerate the affected part with the transplanted mesenchymal stem cells, the population needs to include undifferentiated mesenchymal stem cells to some extent.

In consideration of these points, the culture period is preferably 5 days or more, more preferably 7 days or more, and still more preferably 10 days or more.

In addition, the culture period is preferably 28 days or less, more preferably 21 days or less, and still more preferably 17 days or less.

It is known that mesenchymal stem cells are differentiated into cartilage cells by culturing them in a culture medium to which transforming proliferation factor β3 (TGF-β3), dexamethasone, or bone morphogenetic protein 2 (BMP-2) has been added, whereby the cartilage tissue can be prepared in vitro.

Accordingly, from the viewpoint of suppressing the differentiation of mesenchymal stem cells into cartilage cells, it is preferable that the culture medium does not contain TGF-β3, dexamethasone, and BMP-2.

It is also known that in the mesenchymal stem cells, the in situ cartilage forming ability decreases in inverse proportion to the number of passages of the mesenchymal stem cells in vitro.

Therefore, it is preferable that the mesenchymal stem cells to be produced are primary or first passage mesenchymal stem cells.

In a case where the mesenchymal stem cells after the enzyme treatment are seeded in a culture medium, they are preferably seeded to a cell density of 100 cells/$cm^2$ to 5,000 cells/$cm^2$, more preferably seeded to a cell density of 200 cells/$cm^2$ to 4,500 cells/$cm^2$, still more preferably seeded to a cell density of 300 cells/$cm^2$ to 2,500 cells/$cm^2$, and particularly preferably seeded to a cell density of 400 cells/$cm^2$ to 2,000 cells/$cm^2$.

From the viewpoint of suitability as a therapeutic agent for arthrosis, the number of cells to be recovered at the end of the culture is preferably $0.5 \times 10^7$ cells, more preferably $1.0 \times 10^7$ cells or more, and still more preferably $2.0 \times 10^7$ cells.

In addition, the number of cells to be recovered at the end of the culture may be $3.0 \times 10^7$ cells or more, may be $4.0 \times 10^7$ cells or more, may be $5.0 \times 10^7$ cells or more, and may be $6.0 \times 10^7$ cells or more.

Hereinafter, the mesenchymal stem cells that are used in the production method for a therapeutic agent for arthrosis according to the present disclosure will be described; however, the present disclosure is not limited thereto.

An organism from which a mesenchymal stem cell is derived is not particularly limited; however, the mesenchymal stem cell is preferably a cell derived from a mammalian animal, more preferably a cell derived from a primate animal, and particularly preferably a cell derived from a human.

The mesenchymal stem cells can be obtained by subjecting a biological tissue to an enzyme treatment.

Examples of the biological tissue include synovial tissue, bone marrow tissue, periosteal tissue, adipose tissue, and muscle tissue. Among these, a mesenchymal stem cell (a synovium-derived mesenchymal stem cell) that is obtained by subjecting the synovial tissue to an enzyme treatment is preferable from the viewpoint that the therapeutic agent for arthrosis to be produced is particularly useful for arthrosis as a therapeutic agent for a meniscus, a therapeutic agent for osteoarthritis, and the like.

For example, the synovial tissue can be collected from the unloaded portion of the joint under anesthesia.

Whether or not a cell is the synovium-derived mesenchymal stem cell can be determined, for example, by checking the presence or absence of expression of a cell surface marker and the differentiation potency to cartilage.
The synovium-derived mesenchymal stem cell is CD90-positive and CD45-negative and has a differentiation potency to cartilage.
The biological tissue may be a biological tissue derived from a single donor or may be a biological tissue derived from a plurality of donors; however, it is preferably a biological tissue derived from a single donor.
In a case of producing synovium-derived mesenchymal stem cells for the intended purpose of transplantation to a human, it is preferable to use a biological tissue collected from a donor whose histocompatibility antigen type is identical or similar to that of the patient.
More preferably, a subject from which the synovium is collected and a subject to which the synovium-derived mesenchymal stem cell is transplanted are the same subject. That is, it is preferable to use the synovial tissue collected from the patient himself (autologous transplantation).
The amount of the biological tissue to be collected is preferably changed as appropriate in consideration of the kind of donor and the required amount of mesenchymal stem cells.
For example, the amount to be collected is preferably 0.10 g to 10.00 g, more preferably 0.20 g to 5.00 g, and still more preferably 0.30 g to 4.50 g.
The collected biological tissue is shredded with scissors or the like as necessary, and then subjected to the enzyme treatment described below.
The enzyme is not particularly limited as long as it is an enzyme including a protease; however, it is preferably a mixed enzyme including at least one kind of collagenase and at least one kind of neutral protease from the viewpoint of the digestion rate in a biological tissue.
A particularly preferred enzyme is liberase. As the liberase, it is possible to use, for example, Liberase MNP-S (manufactured by F. Hoffmann-La Roche, Ltd.), which is an enzyme including a collagenase class I, a collagenase class II, and a neutral protease (thermolysin).
The enzymatic reaction can be carried out in an aqueous solution containing an enzyme, and an aqueous solution containing human serum may be used.
The human serum may be an autologous serum or an allogeneic serum; however, it is preferably an autologous serum.
The enzyme concentration in the aqueous solution is preferably 0.01 mg/ml to 10 mg/ml, more preferably 0.1 mg/ml to 10 mg/ml, still more preferably 0.5 mg/ml to 10 mg/ml, even still more preferably 0.5 mg/ml to 5.0 mg, particularly preferably 0.5 mg/ml to 2.0 mg/ml, and most preferably 0.7 mg/ml to 2.0 mg/ml.
In the treatment of the biological tissue with an enzyme, the ratio of the using amount of the biological tissue to the using amount of the enzyme (using amount of biological tissue/using amount of enzyme) is preferably 10 to 1,000, more preferably 20 to 500, and still more preferably 40 to 200 on the mass basis.
The enzymatic reaction temperature is preferably 15°C to 45°C, more preferably 20°C to 43°C, and still more preferably 25°C to 40°C.
The enzymatic reaction time is preferably 45 minutes to 180 minutes, more preferably 60 minutes to 180 minutes, still more preferably 90 minutes to 180 minutes, and particularly preferably 120 minutes to 180 minutes.
A mixture (hereinafter, also referred to as tissue digestion solution) obtained by subjecting a biological tissue to an enzyme treatment contains mesenchymal stem cells, and the mesenchymal stem cells can be recovered by being transferred to a centrifuge tube through a cell strainer and subjected to a centrifugation treatment.
The centrifugation conditions are not particularly limited, which can be set to, for example, a centrifugal force of 200 g to 600 g and a centrifugation time of 1 minute to 10 minutes.
The mixture obtained by the enzyme treatment may be washed once or more. It is preferable to carry out the washing a plurality of times, and it is more preferable to carry out the washing twice.
The washing can be carried out by suspending, in a culture medium, the mesenchymal stem cells which have been recovered by the above-described centrifugation treatment and centrifuging the suspended cells again.
The centrifugation conditions are not particularly limited, which can be set to, for example, a centrifugal force of 200 g to 600 g and a centrifugation time of 1 minute to 10 minutes.
The culture medium is not particularly limited, and it is possible to use, for example, Minimum Essential Medium Eagle Alpha Modification ($\alpha$-MEM).
The washing of the mixture is preferably carried out until the residual enzyme concentration in the supernatant is 0.5 ng/mL or less, more preferably until it is 0.3 ng/mL or less, still more preferably until it is 0.2 ng/mL or less, and particularly preferably until it is 0.1 ng/mL or less.

(Acquisition of cell image, derivation of density information, and detection of colony)

In the production method for a therapeutic agent for arthrosis according to the present disclosure, a cell image of a plurality of mesenchymal stem cells being cultured is acquired, the cell image is analyzed to derive density information on the mesenchymal stem cells in the cell image, colonies of the mesenchymal stem cells in the population are detected based on the derived density information, and the population is sorted based on at least one of an average colony size or a colony forming unit, which is derived from the detected colonies.

An acquisition method for the cell image is not particularly limited and can be carried out with an imaging device known in the related art.

It is preferable that the derived density information is an estimated density distribution of the mesenchymal stem cells in the cell image, which is derived from kernel density estimation.

In addition, It is preferable that the detection of colonies is carried out by carrying out a mean-shift with reference to the estimated density distribution and detecting, as the colony, a collection of the mesenchymal stem cells, which converges to the same maximal point in the estimated density distribution.

The acquisition of the cell image, the derivation of the density information, and the detection of colonies can be carried out by using a cell image analysis device including at least one processor.

Hereinafter, one embodiment (also referred to as a first embodiment) of the cell image analysis device will be described with reference to Fig. 1 to Fig. 22.

According to the production method for a therapeutic agent for arthrosis using the following cell image analysis device, it is not necessary to use a time-lapse image. As a result, it is not necessary to prepare a dedicated device, and thus it is possible to reduce the cost. In addition, since it is not necessary to use a time-lapse image, it is possible to shorten the time taken for acquiring an image. Further, since it is not necessary to use a time-lapse image, it is possible to suppress the analysis load and the cost of data storage.

In Fig. 1, a cell image analysis device 10 is, for example, a desktop personal computer, to which an imaging device 11 is connected. The imaging device 11 is, for example, a phase contrast microscope or a bright field microscope, and it images mesenchymal stem cells 13 being cultured in a culture vessel 12. Then, a cell image 14 obtained with the imaging device 11 is transmitted to the cell image analysis device 10.

A population including an appropriate number of mesenchymal stem cells are seeded in a culture medium. Then, in the population, the mesenchymal stem cells proliferate over time and form colonies. The cell image analysis device 10 detects colonies from the cell image 14.

A method of culturing mesenchymal stem cells will be described below.

In Fig. 2, a computer constituting the cell image analysis device 10 includes a storage device 30, a memory 31, a central processing unit (CPU) 32, a communication unit 33, a display 34, and an input device 35. These components are connected to each other through a bus line 36.

The storage device 30 is a hard disk drive that is built in the computer that constitutes the cell image analysis device 10 or is connected to the computer through a cable or a network. Alternatively, the storage device 30 is a disk array in which a plurality of hard disk drives are connectively mounted. The storage device 30 stores a control program such as an operating system, various application programs, various types of data associated with these programs, and the like. It is noted that a solid state drive may be used instead of the hard disk drive.

The memory 31 is a work memory that is used in a case where the CPU 32 executes processing. The CPU 32 loads the program stored in the storage device 30 into the memory 31 and executes processing according to the program, thereby comprehensively controlling each of the units of the computer.

The communication unit 33 is a network interface that controls the transmission of various information via a network such as a local area network (LAN). The display 34 displays various screens. The computer that constitutes the cell image analysis device 10 receives an input of an operation instruction from the input device 35, through the various screens. The input device 35 is a keyboard, a mouse, a touch panel, or the like.

In Fig. 3, an operation program 40 is stored in the storage device 30 of the cell image analysis device 10. The operation program 40 is an application program for making a computer function as the cell image analysis device 10.

The storage device 30 also stores the cell image 14, a designated band width 41, a detection result 42 of colonies, and an evaluation information 43 indicating the proliferative ability of the mesenchymal stem cells 13.

In a case where the operation program 40 is activated, the CPU 32 of the computer constituting the cell image analysis device 10 cooperates with the memory 31 and the like to function as a read and write (hereinafter, abbreviated as RW) control unit 50, an instruction receiving unit 51, a density information derivation unit 52, a detection unit 53, an evaluation information derivation unit 54, and a display control unit 55. The CPU 32 is an example of the "processor".

An RW control unit 50 controls the storage of various data in the storage device 30 and the reading-out of various data in the storage device 30. For example, the RW control unit 50 receives the cell image 14 from the imaging device 11 and stores it in the storage device 30. In addition, the RW control unit 50 reads out the cell image 14 from the storage device 30 and outputs it to the density information derivation unit 52. That is, the RW control unit 50 is an example of the "acquisition unit".

The instruction receiving unit 51 receives the user’s designation of a band width BW of the kernel density estimation carried out in the density information derivation unit 52 through the input device 35. The instruction receiving unit 51 outputs the received band width BW to the RW control unit 50 as the designated band width 41. The RW control unit 50 stores the designated band width 41 in the storage device 30. In addition, the RW control unit 50 reads out the designated band width 41 from the storage device 30 and outputs it together with the cell image 14 to the density information derivation unit 52.

The designated band width 41 is designated for each culture project of the mesenchymal stem cells 13 generated based on the cells collected from a certain patient. Therefore, in one culture project, the designated band width 41 once designated is repeatedly used throughout the culture project.

The density information derivation unit 52 analyzes the cell image 14 to derive the density information on the mesenchymal stem cells 13 in the cell image 14.

More specifically, the density information derivation unit 52 derives an estimated density distribution 60 of the mesenchymal stem cells 13 in the cell image 14 by carrying out the kernel density estimation with the designated band width 41.

The density information derivation unit 52 outputs the derived estimated density distribution 60 to the detection unit 53 as density information. The density information derivation unit 52 is an example of the "derivation unit".

The detection unit 53 detects colonies of the mesenchymal stem cells 13 based on the estimated density distribution 60. The detection unit 53 outputs the detection result 42 of colonies to the RW control unit 50 and the evaluation information derivation unit 54. The RW control unit 50 stores the detection result 42 in the storage device 30. The detection result 42 is stored in association with the cell image 14.

The evaluation information derivation unit derives evaluation information based on the detection result. The evaluation information derivation unit outputs the evaluation information to the RW control unit. The RW control unit stores the evaluation information in the storage device. The evaluation information 43 is stored in association with the cell image 14 in the same manner as in the detection result 42.

The RW control unit 50 reads out a set of the cell image 14, the detection result 42, and the evaluation information 43, which are stored in association with each other, from the storage device 30 and outputs the set to the display control unit 55.

The display control unit 55 carries out control to display various screens on the display 34. The various screens include a cell image display screen 80 (see Fig. 11) that displays the cell image 14, an analysis result display screen 85 (see Fig. 12) that displays the cell image 14, the detection result 42, and the evaluation information 43, and the like.

In Fig. 4, the density information derivation unit 52 has a cell centroid position extraction unit 65 and a kernel density estimation unit 66. The cell centroid position extraction unit 65 subjects the cell image 14 to binarization processing.

In the binarization processing, first, a threshold value of the brightness value, by which the mesenchymal stem cells 13 in the cell image 14 are separated from the other regions, is set. Then, each of pixel values of the cell image 14 is replaced as follows: in a case where the brightness value is less than the threshold value, the pixel value is replaced with 0, assuming that the corresponding pixel is in a region of the mesenchymal stem cells 13, or in a case where the brightness value is more than the threshold value, the pixel value is replaced with 1, assuming that the corresponding pixel is in a region other than the region of the mesenchymal stem cells 13.

After the binarization processing, the cell centroid position extraction unit 65 subjects the region where the pixel value has been replaced with 0 as the mesenchymal stem cell 13, to a well-known image recognition processing of recognizing each of the mesenchymal stem cells 13. Then, a centroid 68 (indicated by a cross mark) of each of the mesenchymal stem cells 13 recognized in this manner is extracted. The cell centroid position extraction unit 65 outputs the extraction result of the centroid 68 to the kernel density estimation unit 66. Specifically, the extraction result of the centroid 68 is represented on XY coordinates of the centroid 68 in a case where an axis along the long side of the cell image 14 is defined as the X axis, an axis along the short side thereof is defined as the Y axis, and the upper left corner of the cell image 14 is set as the origin.

The kernel density estimation unit 66 estimates the kernel density with the centroid 68 as a sample point and outputs the estimated density distribution 60 as a result of the estimation. More specifically, as shown in Fig. 5, the kernel density estimation unit 66 uses, as a kernel function, a Gaussian function shown by a broken line. The Gaussian function has the designated band width of 41. The kernel density estimation unit 66 assigns a Gaussian function to each of the plurality of centroids 68 and adds (convolutes) a plurality of assigned Gaussian functions to calculate the estimated density distribution 60. The estimated density distribution 60 also includes information on the centroid 68. It is noted that as the kernel function, a rectangular function or a triangular function may be used instead of the Gaussian function.

As shown in Fig. 6 and Fig. 7, the detection unit 53 carries out a mean-shift with reference to the estimated density distribution.

For the mean-shift, a search circle 70 having a radius R centered on each centroid 68 is used. In the mean-shift, the search circle 70 is sequentially moved in a direction in which the density of the mesenchymal stem cells 13 is high, starting from a certain centroid 68 and using the estimated density distribution 60 as a clue, and finally, a maximal point 71 of the density of the mesenchymal stem cell 13, at which the centroid 68 converges, is searched for.

The detection unit 53 subjects each centroid 68 to the mean-shift. The detection unit 53 detects, as a colony, a collection of the mesenchymal stem cells 13 where the centroid 68 has converged to the same maximal point 71 in the estimated density distribution 60.

Fig. 6 and Fig. 7 illustrate an example in which a collection of the mesenchymal stem cells 13 where the centroid 68 has converged to a maximal point 71_1 and a collection of the mesenchymal stem cells 13 where the centroid 68 has converged to a maximal point 71_2 are detected as colonies, respectively.

As illustrated in Fig. 8, the detection unit 53 assigns for each detected colony, a number such as No. 1, No. 2, and so on. In addition, the detection unit 53 generates a rectangular frame 75 circumscribing the centroids 68 of the mesenchymal stem cells 13 for each detected colony. Then, it outputs, together with the number, the XY coordinates of diagonal points PA and PB of the respective upper left corner and lower right corner of the rectangular frame 75, as the detection result 42. Fig. 8 exemplarily illustrates the detection result 42 including diagonal points PA_1 and PB_1 of a rectangular frame 75_1 of a colony No. 1 and diagonal points PA_2 and PB_2 of a rectangular frame 75_2 of a colony No. 2.

As illustrated in Fig. 9, the evaluation information derivation unit 54 derives, as the evaluation information 43, the number of colonies detected by the detection unit 53, the colony forming unit (CFU), and the average colony size.

As described above, the evaluation information 43 is information regarding at least one of the number of colonies, the CFU, or the average colony size at one time point.

In addition, the dilution factor of the culture solution and the amount of the seeded mesenchymal stem cells 13 are input by a user through the input device 35.

As illustrated in Fig. 10, the size of the colony is a value obtained by adding a width WX in the X axis direction and a width WY in the Y axis direction of the rectangular frame 75 and dividing the added value by 2. The average colony size is a value obtained by dividing the integrated value of the colony sizes calculated in this way, by the number of colonies.

Fig. 11 illustrates the cell image display screen 80 that is displayed on the display 34 under the control of the display control unit 55. The cell image 14 in response to a request from a user is displayed on the cell image display screen 80.

An analysis button 81 is provided at the lower part of the cell image display screen 80. In a case of desiring to carry out the image analysis of the cell image 14, a user selects the analysis button 81. In a case where the analysis button 81 is selected, an image analysis instruction is received by the instruction receiving unit 51. As a result, the derivation of the estimated density distribution 60 by the density information derivation unit 52, the detection of colonies by the detection unit 53, and the derivation of the evaluation information 43 the evaluation information derivation unit 54 are carried out.

Fig. 12 illustrates the analysis result display screen 85 that is displayed on the display 34 under the control of the display control unit 55. On the analysis result display screen 85, the cell image 14 on which the number and the rectangular frame 75 are superimposed is displayed for each colony detected by the detection unit 53. That is, the detection result 42 of colonies is presented to a user. The evaluation information 43 is displayed at the lower part of the cell image 14. That is, the evaluation information 43 is presented to a user.

A confirmation button 86 is provided at the lower part of the analysis result display screen 85. In a case where the confirmation button 86 is selected, a display erasure instruction is received by the instruction receiving unit 51. In this case, the display control unit 55 erases the display of the analysis result display screen 85.

Fig. 13 to Fig. 16 illustrates a series of processing for designating the designated band width 41. First, as illustrated in Fig. 13, in the density information derivation unit 52, the cell centroid position extraction unit 65 extracts the centroid 68 of the mesenchymal stem cell 13 in a specific region 90 of a part of the cell image 14. The specific region 90 is a region in the rectangular frame, the size of which is half of the size of the cell image 14 and the center of which coincides with the center of the cell image 14.

The kernel density estimation unit 66 subjects the specific region 90 to a kernel density estimation using a Gaussian function of a band width BW_L, a kernel density estimation using a Gaussian function of a band width BW_M, and a kernel density estimation using a Gaussian function of a band width BW_S. That is, the kernel density estimation unit 66 carries out the kernel density estimation with a plurality of kinds of band widths BW.

The band widths BW_L, BW_M, and BW_S have a magnitude relationship represented by Expression (1) below.

$$BW_L > BW_M > BW_S \cdots (1)$$

That is, the band width BW_L is the largest, and the band width BW_S is the smallest. In addition, the band width BW_M has a size between the band widths BW_L and BW_S. The kind of the band width BW is not limited to three kinds. Two kinds may be used, or four or more kinds may be used.

The kernel density estimation unit 66 derives each of an estimated density distribution 60_L matching with the band width BW_L, an estimated density distribution 60_M matching with the band width BW_M, and an estimated density distribution 60_S matching with the band width BW_S.

That is, the kernel density estimation unit 66 derives a plurality of kinds of the estimated density distributions 60. The estimated density distribution 60_L has the smoothest shape, and the estimated density distribution 60_S has the sharpest shape. The estimated density distribution 60_M has an intermediate shape between the estimated density distributions 60_L and 60_S.

As illustrated in Fig. 14, the detection unit 53 subjects the specific region 90 to a mean-shift with reference to each of the estimated density distributions 60_L, 60_M, and 60_S, to output detection results 42 of colonies which, respectively match with the estimated density distributions 60_L, 60_M, and 60_S.

More specifically, the detection unit 53 outputs, as the detection result 42, XY coordinates of diagonal points PA_L and PB_L of a rectangular frame 75_L (indicated by a two-dot chain line) of colonies detected by carrying out a mean-shift with reference to the estimated density distribution 60_L.

In addition, the detection unit 53 outputs, as the detection result 42, XY coordinates of diagonal points PA_M and PB_M of a rectangular frame 75_M (indicated by a solid line) of colonies detected by carrying out a mean-shift with reference to the estimated density distribution 60_M.

Further, the detection unit 53 outputs, as the detection result 42, XY coordinates of diagonal points PA_S and PB_S of a rectangular frame 75_S (indicated by a broken line) of colonies detected by carrying out a mean-shift with reference to the estimated density distribution 60_S.

The rectangular frame 75_L is relatively large, and the rectangular frame 75_S is relatively small. The rectangular frame 75_M has an intermediate size between the rectangular frames 75_L and 75_S. These detection results 42 are output to the display control unit 55.

The display control unit 55 displays a designation screen 95 illustrated in Fig. 15 on the display 34. The specific region 90, on which the rectangular frames 75_L, 75_M, and 75_S as the detection results 42 are superimposed, is displayed on the designation screen 95. The rectangular frames 75_L, 75_M, and 75_S are displayed in different colors (for example, the rectangular frame 75_L is blue, the rectangular frame 75_M is yellow, and the rectangular frame 75_S is green).

At the lower part of the specific region 90, frame designation buttons 96L, 96M, and 96S are provided together with a message indicating that a user is instructed to designate the rectangular frame 75 that is conceived to be suitable as the colony. The frame designation button 96L matches with the rectangular frame 75_L, the frame designation button 96M matches with the rectangular frame 75_M, and the frame designation button 96S matches with the rectangular frame 75_S. The frame designation buttons 96L, 96M, and 96S are alternative buttons, and thus in a case where one is selected, the other two cannot be selected. That is, the display control unit 55 presents a plurality of kinds of the detection results 42 to a user and allows the user to select one of the plurality of kinds of the detection results 42. A designation button 97 is further provided at the lower part of the frame designation buttons 96L, 96M, and 96S.

As illustrated in Fig. 16, in a case where the designation button 97 is selected in a state where one of the frame designation buttons 96L, 96M, and 96S is selected, the instruction receiving unit 51 receives the designation of the band width BW. The instruction receiving unit 51 sets a band width BW matching with one frame designation button 96 selected by a user as the designated band width 41. That is, The instruction receiving unit 51 sets a band width BW matching with one detection result 42 selected by a user as the designated band width 41. Fig. 16 exemplarily illustrates a case where the frame designation button 96M is selected by a user and the band width BW_M is set as the designated band width 41.

Next, the actions of the above-described configuration will be described with reference to flowcharts of Fig. 17 and Fig. 18.

First, in a case where the operation program 40 is activated in the cell image analysis device 10, the CPU 32 of the cell image analysis device 10 functions as the RW control unit 50, the instruction receiving unit 51, the density information derivation unit 52, the detection unit 53, the evaluation information derivation unit 54, and the display control unit 55, as illustrated in Fig. 3.

The RW control unit 50 reads out the cell image 14 in response to a request from a user, from the storage device 30 (a step ST100). The cell image 14 is output from the RW control unit 50 to the display control unit 55.

Under the control of the display control unit 55, the cell image display screen 80 illustrated in Fig. 11 is displayed on the display 34 (a step ST110). In this way, the cell image 14 is presented to a user.

In a case where the analysis button 81 is selected by a user, the image analysis instruction is received by the instruction receiving unit 51 (YES in the step ST120), and the designated band width 41 is already stored in the storage device 30 (YES in the step ST130), the RW control unit 50 reads out the cell image 14 and the designated band width 41 from the storage device 30 (a step ST140). The cell image 14 and the designated band width 41 are output from the RW control unit 50 to the density information derivation unit 52. It is noted that step ST140 is an example of the "acquisition step".

As shown in Fig. 4 and Fig. 5, the density information derivation unit 52 carries out the kernel density estimation with the designated band width 41. As a result, the estimated density distribution 60 is derived as the density information (a step ST150). The estimated density distribution 60 is output from the density information derivation unit 52 to the detection unit 53. The step ST150 is an example of the "derivation step".

As shown in Fig. 6 and Fig. 7, the detection unit 53 carries out a mean-shift with reference to the estimated density distribution 60. As a result, colonies of the mesenchymal stem cells 13 in the cell image 14 are detected (a step ST160). The detection result 42 of colonies is output from the detection unit 53 to the RW control unit 50 and stored in the storage device 30. In addition, the detection result 42 of colonies is output from the detection unit 53 to the evaluation information derivation unit 54. The step ST 160 is an example of the "detection step".

As illustrated in Fig. 9, in the evaluation information derivation unit 54, the number of colonies, the colony forming unit, and the average colony size are derived as the evaluation information 43 based on the detection result 42 (a step ST170). The evaluation information 43 is output from the evaluation information derivation unit 54 to the RW control unit 50 and stored in the storage device 30.

The detection result 42 and the evaluation information 43 are read out from the storage device 30 by the RW control unit 50 and output to the display control unit 55. In addition, under the control of the display control unit 55, the analysis result display screen 85 illustrated in Fig. 12 is displayed on the display 34 (a step ST180). As a result, the detection result 42 and the evaluation information 43 are presented to a user. The display of the analysis result display screen 85 is erased in a case where the confirmation button 86 is selected and the display erasure instruction is received by the instruction receiving unit 51 (YES in the step ST190). Then, the processing is terminated.

It is noted that in a case where the instruction receiving unit 51 does not receive the image analysis instruction (NO in the step ST120) and does not receive the termination instruction (NO in the step ST200), the display of the cell image display screen 80 is continued. In a case where the instruction receiving unit 51 receives the termination instruction (YES in the step ST200), the display of the cell image display screen 80 is erased, and the processing is terminated.

In a case where the designated band width 41 is not stored in the storage device 30 (NO in the step ST130), the processing illustrated in Fig. 18 is carried out.

Specifically, first, as illustrated in Fig. 13, the density information derivation unit 52 subjects the specific region 90 to the kernel density estimation with three kinds of band widths BW_L, BW_M, and BW_S, thereby respectively deriving three kinds of estimated density distributions 60_L, 60_M, and 60_S (a step ST1301).

Subsequently, as illustrated in Fig. 14, the detection unit 53 subjects the specific region 90 to a mean-shift with reference to each of three kinds of the estimated density distributions 60_L, 60_M, and 60_S, thereby outputting three kinds of the detection results 42 of colonies, which respectively match with three kinds of the estimated density distributions 60_L, 60_M, and 60_S (a step ST1302).

Specifically, the three kinds of the detection results 42 of colonies are the XY coordinates of the diagonal points PA_L and PB_L of the rectangular frame 75_L, the diagonal points PA_M and PB_M of the rectangular frame 75_M, and the diagonal points PA_S and PB_S of the rectangular frame 75_S.

In addition, as illustrated in Fig. 15, the designation screen 95 is displayed on the display 34 under the control of the display control unit 55 (a step ST1303). On the designation screen 95, the frame designation buttons 96L, 96M, and 96S, which respectively match with three kinds of the rectangular frames 75_L, 75_M, and 75_S are provided to be alternatively selectable.

As illustrated in Fig. 16, a user selects the frame designation button 96 matching with the rectangular frame 75 that is conceived to be suitable as the colony, and selects the designation button 97. As a result, the instruction receiving unit 51 receives the designation of the band width BW (a step ST1304). The band width BW matching with the frame designation button 96 selected by the user is set as the designated band width 41 by instruction receiving unit 51. The designated band width 41 is output from the instruction receiving unit 51 to the RW control unit 50 and stored in the storage device 30 by the RW control unit 50 (a step ST1305).

As described above, the cell image analysis device 10 includes the RW control unit 50 as an acquisition unit, the density information derivation unit 52 as a derivation unit, and the detection unit 53. The RW control unit 50 acquires the cell image 14 obtained by imaging the mesenchymal stem cells 13 with the imaging device 11, by reading out the cell image 14 from the storage device 30. The density information derivation unit 52 analyzes the cell image 14 to derive the estimated density distribution 60 as the density information on the mesenchymal stem cells 13 in the cell image 14. The detection unit 53 detects colonies of the mesenchymal stem cells 13 based on the estimated density distribution 60.

The density information derivation unit 52 derives an estimated density distribution 60 of the mesenchymal stem cells 13 in the cell image 14 by carrying out the kernel density estimation and outputs the estimated density distribution 60 as density information. Since the estimated density distribution 60 is derived by a commonly used method called kernel density estimation and this is used as the density information, it is possible to easily obtain the density information that indicates the density of the mesenchymal stem cells 13 relatively accurately.

The detection unit 53 carries out a mean-shift with reference to the estimated density distribution 60 to detect, as the colony, a collection of the mesenchymal stem cells 13, which has converged to the same maximal point 71 in the estimated density distribution 60. The mean-shift is also a method commonly used together with kernel density estimation. Therefore, colonies can be easily detected with a relatively high accuracy. In addition, as compared with the detection of colonies with a visual observation by a user, colonies can be detected without hesitation based on a consistent standard.

The instruction receiving unit 51 receives the user's designation of the band width BW of the kernel density estimation. The density information derivation unit 52 carries out the kernel density estimation with the designated band width 41, which is the band width BW received by the instruction receiving unit 51. Therefore, the validity of the estimated density distribution 60 can be enhanced. In addition, as a result, it is possible to detect a colony that matches a user's feeling.

The density information derivation unit 52 subjects a part of the specific regions 90 of the cell image 14 to the kernel density estimation with a plurality of kinds of band widths BW, thereby deriving a plurality of kinds of the estimated density distributions 60. The detection unit 53 carries out a mean-shift with reference to each of the plurality of kinds of the estimated density distributions 60, thereby outputting the detection results 42 of a plurality of kinds of colonies, which respectively match with the plurality of kinds of the estimated density distributions 60. The display control unit 55 presents a plurality of kinds of the detection results 42 to a user and allows the user to select one of the plurality of kinds of the detection results 42. The instruction receiving unit 51 sets a band width BW matching with one detection result 42 selected by a user as the designated band width 41. A user can select for oneself the detection result 42 that is conceived to be suitable as the colony while comparing the plurality of kinds of the detection results 42. Therefore, it is easy to select the detection result 42.

The display control unit 55 presents the detection result 42 of colonies to a user. Therefore, a user can confirm the detection result 42 of colonies.

The evaluation information derivation unit 54 derives the evaluation information 43 indicating the proliferative ability of the mesenchymal stem cell 13 based on the detection result 42 of colonies. The display control unit 55 presents the evaluation information 43 to a user. Therefore, the user can confirm the evaluation information 43 and can evaluate the proliferative ability of the mesenchymal stem cell 13 with reference to the evaluation information 43.

The evaluation information 43 is information regarding at least any of the number of colonies, the CFU, or the average colony size at one time point. Therefore, a user can evaluate the proliferative ability of the mesenchymal stem cell 13 at one time point.

In addition, it is possible to set the detection unit 53 so that the derived evaluation information 43 is limited to the information on the mesenchymal stem cell 13 having a colony size exceeding a certain size.

Specifically, the detection unit 53 can be set to derive an average colony size for a colony having a colony size of more than 0.54 mm.

It is noted that the evaluation information may include evaluation information other than the number of colonies, the CFU, and the average colony size, which are exemplarily illustrated in Fig. 9.

For example, the evaluation information may be an average value of the number of the mesenchymal stem cells 13 constituting a colony, an average value of a ratio (WX/WY) of the width WX to the width WY of the rectangular frame 75, or the like.

It can be said that the closer the ratio of the width WX to the width WY of the rectangular frame 75 is to 1, that is, the closer the rectangular frame 75 is to a square, the more uniformly the mesenchymal stem cells 13 constituting a colony proliferates, which is preferable.

In addition, as in an evaluation information 100 illustrated in Fig. 19, statistical information such as a histogram 101, in which the frequency on the vertical axis is for the number of colonies and the class on the horizontal axis is for sizes of colonies, may be included. A reference numeral 102 is a line segment indicating the average colony size.

In addition, the evaluation information is not limited to the information at one time point but may be information at a plurality of time points.

For example, as in an evaluation information 105 illustrated in Fig. 20, a graph 106 showing a change in the average colony size over time with the average colony size as the vertical axis and the number of days of culture of the mesenchymal stem cells 13 as the horizontal axis may be included.

The graph 106 displays a plot 107 (indicated by ×) of the average colony size for each number of days of culture, an approximate straight line 108 of each plot 107, and a text balloon 109. The amount of change in the average colony size per number of days of culture is recorded in the text balloon 109.

In addition, a threshold value may be set for the amount of change in average colony size. Then, in a case where the amount of change in average colony size is less than the threshold value, a warning 110 may be displayed as illustrated in Fig. 21. The threshold value is a value set from an empirical point of view, and it is a value at which it is clear that the quality of the mesenchymal stem cells 13 after the completion of the culture does not reach the shippable level. This makes it possible to urge a user to take various measures such as stopping the culture of the mesenchymal stem cells 13 or raising the quality of the mesenchymal stem cells 13 to the shipping level, for example, by changing a component of the culture medium.

As a designation method for the designated band width 41, an aspect illustrated in Fig. 22 may be adopted.

First, the band width BW is set to a first band width BW_I (not illustrated in the drawing), which is an initial value (a step ST1310). The first band width BW_I is, for example, the band width BW_L described above.

Next, the density information derivation unit 52 subjects the specific region 90 to the kernel density estimation with the first band width BW_I, thereby deriving an estimated density distribution 60_I (a step ST1311).

Subsequently, the detection unit 53 subjects the specific region 90 to a mean-shift with reference to the estimated density distribution 60_I, thereby outputting the detection result 42 of colonies, which matches with the estimated density distribution 60_I (a step ST1312). Specifically, the detection result 42 of colonies is XY coordinates of diagonal points PA_I and PB_I (not illustrated in the drawing) of a rectangular frame 75_I (not illustrated in the drawing).

Next, under the control of the display control unit 55, the display screen of the cell image 14 on which the rectangular frame 75_I, which is the detection result 42 of colonies, is superimposed is displayed on the display 34 (a step ST1313). The display screen includes a button for allowing a user to select whether or not the region indicated by the rectangular frame 75_I is suitable as the colony.

In a case where a button indicating that the region indicated by the rectangular frame 75_I is not suitable as the colony is selected by a user (NO in the step ST1314), the band width BW is changed from the first band width BW_I to a second band width BW_SE (not illustrated in the drawing) (a step ST1315). The second band width BW_SE is, for example, the band width BW_M according to the first embodiment. Then, the processing of the step ST1311 is carried out with the second band width BW_SE, and further, the processing of the steps ST1312 and ST1313 is also carried out. A series of processing from step ST1315 to step ST1313 are repeated until a button indicating that the region indicated by the rectangular frame 75 is suitable as the colony is selected by a user.

In a case where a button indicating that the region indicated by the rectangular frame 75 is suitable as the colony is selected by a user, (YES in the step ST1314), The band width BW set at that time is set as the designated band width 41 by the instruction receiving unit 51. The designated band width 41 is output from the instruction receiving unit 51 to the RW control unit 50 and stored in the storage device 30 by the RW control unit 50 (a step ST1316).

In this way, a user may be allowed to select whether or not the region indicated by the rectangular frame 75 is suitable as the colony, by stepwise changing the band width BW and presenting the rectangular frame 75 each time as the detection result 42 of colonies to the user. It is noted that a user may be allowed to input the rectangular frame 75 that is conceived to be suitable as the colony, thereby setting the band width BW matching with the input rectangular frame 75 as the designated band width 41.

Another embodiment (hereinafter, also referred to as a second embodiment) of the cell image analysis device will be described with reference to Fig. 23 to Fig. 27.

In the second embodiment, the derivation of the density information and the detection of the colonies are executed only in a case where the index indicating the degree of proliferation of the mesenchymal stem cells 13 in the cell image 14 is within a set range 123.

In Fig. 23 to Fig. 25, the CPU of the cell image analysis device functions as an area ratio calculation unit 120 and a determination unit 121, in addition to the respective processing units 50 to 55 according to the first embodiment.

The area ratio calculation unit 120 calculates an area ratio 122 of the mesenchymal stem cells 13 in the cell image 14. Specifically, the area ratio calculation unit 120 subjects the cell image 14 to binarization processing in the same manner as in the cell centroid position extraction unit 65.

Next, the number of pixels, in which the pixel value is replaced with 0 assuming that the cells are mesenchymal stem cells 13, is counted. Then, the area ratio 122 of the mesenchymal stem cells 13 is calculated by dividing the counted number of pixels by the total number of pixels of the cell image 14. The area ratio calculation unit 120 outputs the calculated area ratio 122 to the determination unit 121. The area ratio 122 is an example of the "index".

The determination unit 121 determines whether or not the area ratio 122 from the area ratio calculation unit 120 is within the set range 123. The set range 123 is stored in the storage device 30, and it is read out from the storage device 30 by the RW control unit 50 delivered to the determination unit 121.

As illustrated in Fig. 23, in a case where the area ratio 122 is within the set range 123, the determination unit 121 outputs a determination result 124A having the content that "the derivation of density information and the detection of colonies are executed".

In this case, the density information derivation unit 52 executes the derivation of the estimated density distribution 60 which is the density information, and the detection unit 53 also executes the detection of colonies.

As illustrated in Fig. 24 and Fig. 25, in a case where the area ratio 122 is outside the set range 123, the determination unit 121 outputs a determination result 124B having the content that "the derivation of density information and the detection of colonies are not executed".

In this case, the density information derivation unit 52 does not execute the derivation of the estimated density distribution 60 which is the density information, and the detection unit 53 also does not execute the detection of colonies.

Fig. 23 to Fig. 25 exemplarily illustrate a case where the set range 123 of the area ratio is set to 25% to 75%.

Fig. 23 illustrates an example in which the area ratio 122 is 41%, which is within the set range 123, and the determination unit 121 outputs the determination result 124A. Fig. 24 illustrates an example in which the area ratio 122 is 14%, which is outside the set range 123, and the determination unit 121 outputs the determination result 124B. Fig. 25 illustrates an example in which the area ratio 122 is 79%, which is outside the set range 123, and the determination unit 121 outputs the determination result 124B.

Fig. 26 is a view summarizing the aspects shown in Fig. 23 to Fig. 25 in a graph in which the vertical axis indicates the area ratio and the horizontal axis indicates the number of days of culture.

The mesenchymal stem cells 13 tend to have a very high proliferation rate, and thus the average colony size thereof reaches a plateau at a relatively early stage.

However, in the initial stage of culture, the cells are not dense, and the number thereof is not enough to be said to form colonies.

Therefore, in a case where the area ratio 122 is less than 25%, which can be said to be the initial stage of culture, the determination unit 121 outputs a determination result 124B having the content that "the derivation of density information and the detection of colonies are not executed".

In addition, in the final stage of culture in which the average colony size reaches the plateau, colonies cover substantially the entire culture vessel 12, and thus it is meaningless to distinguish the colonies. Therefore, even in a case where the area ratio 122 is more than 75%, which can be said to be the final stage of culture, the determination unit 121 outputs a determination result 124B having the content that "the derivation of density information and the detection of colonies are not executed".

Fig. 27 illustrates the cell image display screen 80 in a case where the area ratio 122 is outside the set range 123 and the determination unit 121 outputs the determination result 124B. In this case, when the analysis button 81 is selected, the display control unit 55 carries out pop-up displaying of a warning window 130. A message indicating that analysis cannot be carried out is displayed in the warning window 130 since the area ratio 122 is outside the set range 123. The display of the warning window 130 disappears by selecting the OK button 131.

In this way, in the second embodiment, the derivation of the density information and the detection of the colonies are executed only in a case where the area ratio 122 which is an index indicating the degree of proliferation of the mesenchymal stem cells 13 in the cell image 14 is within a set range 123.

As a result, it is possible to avoid an unnecessary image analysis in a case where the execution of the image analysis is meaningless, such as a case of the initial stage of culture (a stage in which the mesenchymal stem cells 13 do not form colonies) or the final stage of culture (a stage in which the mesenchymal stem cells 13 form large colonies over substantially the entire culture vessel 12). In other words, the image analysis can be executed only in a period suitable for the evaluation of the proliferative ability of the mesenchymal stem cell 13.

It is noted that the index indicating the degree of proliferation of the mesenchymal stem cells 13 in the cell image 14 is not limited to the exemplary area ratio 122. It may be the number of the mesenchymal stem cells 13 in the cell image 14.

Still another embodiment (hereinafter, also referred to as a third embodiment) of the cell image analysis device will be described with reference to Fig. 28 to Fig.30.

In Fig. 28, the density information derivation unit 140 derives, as density information, the number 142 of the mesenchymal stem cells 13 in the search frame 141 set in advance (hereinafter, referred to as the number of in-frame cells). The search frame 141 has, for example, the same size as that of the rectangular frame 75_M according to the first embodiment. Alternatively, the search frame 141 is set by a user. The number 142 of in-frame cells is obtained, for example, by counting the number of the centroids 68 of the mesenchymal stem cells 13 present in the search frame 141. The density information derivation unit 140 outputs the number 142 of in-frame cells to the detection unit 143.

The detection unit 143 detects colonies, based on the number 142 of in-frame cells and a colony condition 144, and it outputs the detection result 145. The colony condition 144 is stored in the storage device 30, and it is read out from the storage device 30 by the RW control unit 50 delivered to the detection unit 143.

As illustrated in Fig. 29, the density information derivation unit 140 sequentially scans a plurality of regions RE1_1, RE1_2, ..., RE2_1, ..., and REM_N in the cell image 14 with the search frame 141, and it derives the number 142 of in-frame cells in each of the regions.

The detection unit 143 detects whether or not each region RE is a colony region, depending on whether or not the number 142 of in-frame cells in each region RE satisfies the colony condition 144. It is noted that the region RE1_2 is a region in which the search frame 141 has been half-shifted from the position thereof in the region RE1_1 in the X axis direction. In addition, the region RE2_1 is a region in which the search frame 141 has been half-shifted from the position thereof in the region RE1_1 in the Y axis direction.

For example, as illustrated in Fig. 30, in a case where the number 142 of in-frame cells in the region RE2_1 is 52 and the colony condition 144 is such that the number of in-frame cells is 50 or more, the detection unit 143 detects the region RE2_1 as a colony region.

In this way, in the third embodiment, the number 142 of in-frame cells is derived as the density information, instead of the estimated density distribution 60. Therefore, the density information can be easily derived as compared with the estimated density distribution 60.

The search frame 141 is not limited to one kind. A plurality of kinds of the search frames 141 having sizes, shapes, or the like different from each other may be set, and colonies may be detected for each search frame 141.

Still another embodiment (hereinafter, also referred to as a fourth embodiment) of the cell image analysis device will be described with reference to Fig. 31 and Fig.32.

In the fourth embodiment, the image analysis is carried out using a machine learning model 150.

In Fig. 31, the machine learning model 150 is a model in which the cell image 14 is used as an input image and the cell image 14 on which the rectangular frame 75 as the detection result 42 of colonies is superimposed is used as an output image. The machine learning model 150 is, for example, a convolutional neural network such as U-Shaped Neural Network (U-Net), SegNet, Residual Network (ResNet), or Densely Connected Convolutional Network (DenseNet).

Fig. 32 illustrates an outline of processing in a learning phase of the machine learning model 150. In the learning phase, learning data 155 is provided to train the machine learning model 150. The learning data 155 is a set of a cell image for learning 14L and a correct answer image 14CA matching with the cell image for learning 14L. The correct answer image 14CA is an image in which the rectangular frame 75 is manually drawn by a user in a region of the cell image for learning 14L, which is conceived to be the colony region.

In the learning phase, the cell image for learning 14L is input to the machine learning model 150. The machine learning model 150 outputs an output image for learning 14OL with respect to the cell image for learning 14L. The output image for learning 14OL is the cell image for learning 14L in which the rectangular frame 75 is superimposed on a region estimated to be a colony region in the machine learning model 150. The operation for loss for the machine learning model 150 is carried out based on the output image for learning 14OL and the correct answer image 14CA. Then, update settings of various coefficients of the machine learning model 150 are made according to the result of the operation for loss, and the machine learning model 150 is updated according to the update settings. In a case where there is a large difference between the rectangular frame 75 of the output image for learning 14OL and the rectangular frame 75 of the correct answer image 14CA, the loss is large and thus the degree of update of the machine learning model 150 is also large. On the contrary, In a case where there is almost no difference between the rectangular frame 75 of the output image for learning 14OL and the rectangular frame 75 of the correct answer image 14CA, the loss is small and thus the degree of update of the machine learning model 150 is also small.

In the learning phase, the above series of processing of inputting the cell image for learning 14L to the machine learning model 150, outputting the output image for learning 14OL from the machine learning model 150, operation for loss, update setting, and updating the machine learning model 150 is repeatedly carried out while the learning data 155 being exchanged.

The repetition of the above series of processing is terminated in a case where the estimation accuracy of the rectangular frame 75 of the output image for learning 14OL reaches a predetermined set level. The machine learning model 150 in which the estimation accuracy of the rectangular frame 75 reaches the set level is stored in the storage device 30 and used.

In this way, in the fourth embodiment, colonies are detected using the machine learning model 150. Therefore, the colonies can be detected more efficiently.

It is noted that, instead of the correct answer image 14CA, the XY coordinates of the diagonal points PA and PB of the rectangular region 75, which is conceived to be a colony in the cell image for learning 14L, may be used as the learning data 155.

The designated band width 41 may be changed for each cell image 14 instead of for each culture project. In addition, the designated band width 41 may be changed depending on the kind of the mesenchymal stem cell 13 (for example, for an A cell, the designated band width 41 is set as the band width BW_M, and for a B cell, the designated band width 41 is set as the band width BW_S).

The detection result 42 of colonies is not limited to the exemplary rectangular frame 75, and it may be, for example, a curved frame (such as a circular or elliptic frame) surrounding a detected colony region.

Similar to the band width BW, the radius R of the search circle 70 that is used in the mean-shift may be designated by a user.

The aspect in which the detection result 42 of colonies is presented to a user is not limited to the aspect in which the cell image 14 on which the rectangular frame 75 is superimposed is displayed on the display 34.

For example, an aspect in which the cell image 14 on which the rectangular frame 75 is superimposed is printed out or an aspect in which the cell image 14 on which the rectangular frame 75 is superimposed is distributed to a terminal owned by a user by e-mail or the like may be adopted.

Similarly, the aspect in which the evaluation information 43 is presented to a user is not limited either to the aspect in which the evaluation information 43 is displayed on the display 34.

For example, an aspect in which the evaluation information 43 is printed out or an aspect in which the evaluation information 43 is distributed to a terminal owned by a user by e-mail or the like may be adopted.

Various modifications may be made to the hardware configuration of the computer that constitutes the cell image analysis device 10. The cell image analysis device 10 may be composed of a plurality of computers separated as hardware for the intended purpose of improving processing capacity and reliability. For example, the functions of the density information derivation unit 52 and the detection unit 53, and the function of the evaluation information derivation unit 54 are respectively distributed to and played by two computers. In this case, the cell image analysis device 10 is composed of two computers.

In this manner, the hardware configuration of the computer of the cell image analysis device 10 may be appropriately modified according to necessary performance such as processing capacity, security, and reliability.

Further, not only the hardware but also the application program such as the operation program 40 can be duplicated and distributed to be stored in a plurality of storage devices for the intended purpose of ensuring safety and reliability.

In each of the above embodiments, for example, as a hardware structure of processing units that execute various processing, such as the RW control unit 50, the instruction receiving unit 51, the density information derivation units 52 and 140, the detection units 53 and 143, the evaluation information derivation unit 54, the display control unit 55, the cell centroid position extraction unit 65, the kernel density estimation unit 66, the area ratio calculation unit 120, and the determination unit 121, the various processors described below can be used.

As described above, in addition to the CPU 32 that is a general-purpose processor that executes software (operation program 40) to function as various processing units, various processors include a programmable logic device (PLD) that is a processor of which a circuit configuration is changeable after manufacturing, such as a field programmable gate array (FPGA), a dedicated electrical circuit that is a processor having a circuit configuration specifically designed to execute a specific process, such as an application specific integrated circuit (ASIC), or the like.

One processing unit may be composed of one of these various processors or a combination of two or more processors of the same type or different types (for example, a combination of a plurality of FPGAs or a combination of a CPU and an FPGA). Further, a plurality of processing units may be composed of one processor.

As an example in which the plurality of processing units is composed of one processor, first, as represented by a computer such as a client and a server, there is a configuration in which one processor is composed of a combination of one or more CPUs and software and this processor functions as a plurality of processing units.

Secondly, as represented by a system on chip (SoC) or the like, there is a configuration in which a processor that realizes the functions of the entire system including a plurality of processing units by one integrated circuit (IC) chip is used.

As described above, one or more of the above various processors are used to constitute the hardware structure of the various processing units.

Further, as a hardware structure of these various processors, more specifically, an electrical circuit (circuitry) in which circuit elements such as semiconductor elements are combined may be used.

The description contents and the illustrated contents shown above are merely examples of the technology of the present disclosure. For example, the above description relating to the configuration, function, operation, and advantageous effects is a description relating to the configuration, function, operation, and advantageous effects of the portions according to the technology of the present disclosure. Accordingly, within the scope without departing from the concept of the technology of the present disclosure, unnecessary portions may be removed, or new elements may be added or replaced for the above-described content and the above-illustrated content.

In addition, in order to avoid complication and facilitate understanding of the portions related to the technology of the present disclosure, in the above-described content and the above-illustrated content, the description of the common technical knowledge or the like that does not need special explanation in implementing the technology of the present disclosure is omitted.

(Sorting of population)

In the production method for a therapeutic agent for arthrosis according to the present disclosure, the population is sorted based on at least one of an average colony size or a colony forming unit, which is derived from the detected colonies.

As a specific example, the population can be sorted based on the evaluation information of the average colony size and the colony forming unit, which is derived from the evaluation information derivation unit.

More specifically, it is preferable that the detection of the colonies is carried out on a 4th day to a 6th day from a culture start date and the sorting of the population is carried out by sorting a population that satisfies at least one of the following condition a1) or b1), it is more preferable that the detection of the colonies is carried out by sorting a population that satisfies at least one of the following condition a1') or b1'), and it is still more preferable that the detection of the colonies is carried out by sorting a population that satisfies at least one of the following condition a1") or b1").

a1) The average colony size of the mesenchymal stem cells included in the population at any time point of the 4th day to the 6th day from the culture start date start date is 0.740 mm or more.

b1) The colony forming unit of the mesenchymal stem cells included in the population at any time point of the 4th day to the 6th day from the culture start date is 0.250 units/mm$^2$ or more.

a1') The average colony size of the mesenchymal stem cells included in the population at any time point of the 4th day to the 6th day from the culture start date start date is 0.750 mm or more.

b1') The colony forming unit of the mesenchymal stem cells included in the population at any time point of the 4th day to the 6th day from the culture start date is 0.270 units/$mm^2$ or more.

a1") The average colony size of the mesenchymal stem cells included in the population at any time point of the 4th day to the 6th day from the culture start date start date is 0.800 mm or more.

b1") The colony forming unit of the mesenchymal stem cells included in the population at any time point of the 4th day to the 6th day from the culture start date is 0.300 units/$mm^2$ or more.

In a population that satisfies at least one of the following condition a1) or b1), it is predicted that the number of mesenchymal stem cells reach a desired number by the day when transplantation is carried out, and a therapeutic agent for arthrosis, which is produced by carrying out such sorting is an agent suitable for the medical treatment of arthritis.

(Others)

A pharmaceutically acceptable carrier may be mixed with synovium-derived mesenchymal stem cells that have been cultured to prepare a suspension or a gel-like substance, which may be used as a therapeutic agent for arthrosis.

In addition, synovium-derived mesenchymal stem cells that have been cultured may be used as a cell sheet or the like for a therapeutic agent for arthrosis without being mixed with a carrier.

Examples of the pharmaceutically acceptable carrier include aqueous carriers such as saline and an isotonic solution containing glucose, D-sorbitol, D-mannitol, sodium chloride, or the like.

In addition, a bioabsorbable gel such as gelatin or collagen may be used as the pharmaceutically acceptable carrier.

The therapeutic agent for arthrosis may contain a pharmaceutically acceptable additive, examples of which include a buffering agent (for example, a phosphate buffer solution or a sodium acetate buffer solution), a stabilizer (for example, human serum albumin or polyethylene glycol), a soothing agent (for example, benzalkonium chloride or procaine hydrochloride), a preservative, and an antioxidant.

(Therapeutic agent for arthrosis)

The therapeutic agent for arthrosis according to the present disclosure is a therapeutic agent for arthrosis that is produced by the above-described production method for a therapeutic agent for arthrosis.

Since the therapeutic agent for arthrosis has been described above, the description thereof will be omitted here.

Hereinafter, an example of a method of using the therapeutic agent for arthrosis (a medical treatment method for arthritis) according to the present disclosure will be described, which is not limited thereto.

The therapeutic agent for arthrosis can be used for the medical treatment for arthrosis by transplanting the therapeutic agent for arthrosis (a suspension containing mesenchymal stem cells, a cell sheet of mesenchymal stem cells, or mesenchymal stem cells which have been made into as a gel-like substance with a carrier such as a gelatin or collagen) so that a cartilage injury part or a meniscus injury part is covered by the mesenchymal stem cells, and differentiating the mesenchymal stem cells into cartilage cells in the injury part to regenerate the cartilage tissue in situ.

During the in situ cartilage formation process of the mesenchymal stem cells, the cartilage tissue is regenerated depending on the local microenvironment (the nutrient supply, the cytokine environment, and the like), and thus no external operation is required. As a result of the in situ cartilage formation of mesenchymal stem cells, the cartilage tissue is regenerated at the cartilage injury part or meniscus injury part to repair the injury. Specifically, in a case of the cartilage injury, a bone region, a boundary between the cartilage and the bone, a central part of the cartilage, a surface region, and a region adjacent to the original cartilage are formed as the original cartilage tissue, or in a case of the meniscus injury, meniscus cartilage is formed.

The transplantation of mesenchymal stem cells can be carried out by exposing a cartilage injury part or a meniscus injury part by a surgical treatment and then covering the injury part with the therapeutic agent for arthrosis.

The method of transplanting the mesenchymal stem cells is not limited to the above method, and it may be carried out, for example, by injecting a suspension containing the mesenchymal stem cells into a joint.

The surgical treatment can be carried out by open surgery or arthroscopic surgery. Arthroscopic surgery is preferable since the invasion can be made as small as possible.

From the viewpoint of the adhesiveness of the mesenchymal stem cells to the injury part, the time taken for covering the injury part with the therapeutic agent for arthrosis is preferably 10 minutes or more and more preferably 15 minutes or more.

In order to further improve the adhesiveness of the mesenchymal stem cells to the injury part, the site covered by the therapeutic agent for arthrosis can be further covered by the periosteum.

In a case of transplanting mesenchymal stem cells to a patient, from the viewpoint of medical treatment efficiency for arthritis, the number of mesenchymal stem cells to be transplanted to the injury part, which are contained in the therapeutic agent for arthrosis, is preferably $0.50 \times 10^7$ or more, more preferably $1.00 \times 10^7$ or more, and still more preferably $2.00 \times 10^7$ or more. In addition, the number of mesenchymal stem cells to be transplanted to the injury part, which are contained in the therapeutic agent for arthrosis, may be $2.50 \times 10^7$ cells or more, may be $3.00 \times 10^7$ cells or more, or may be $4.00 \times 10^7$ cells or more.

In addition, the upper limit value of the number of mesenchymal stem cells to be transplanted to the injury part, which are contained in the therapeutic agent for arthrosis, is not limited thereto; however, it may be, for example, $1.0 \times 10^{11}$ cells or less, may be $1.0 \times 10^{10}$ cells or less, may be $1.0 \times 10^9$ cells or less, or may be $1.0 \times 10^8$ cells or less.

The disclosure of JP2021-040648 filed on March 12, 2021 is incorporated in the present specification by reference in its entirety. All publications, patent applications, and technical standards mentioned in the present specification are herein incorporated by reference to the same extent as in a case where each individual publication, patent application, and technical standard were specifically and individually indicated to be incorporated by reference. Examples

Hereinafter, the above-described embodiment will be specifically described with reference to Examples; however, the above-described embodiment is not limited to these Examples.

<Example 1>

Synovium-derived mesenchymal stem cells were prepared after sterilizing the synovial tissue, by using a human specimen of a patient with a meniscus injury.

Specifically, a tissue of an M1 specimen (0.85 g of the synovial tissue) was shredded with scissors, immersed in 5.0 mL of a liberase aqueous solution, and subjected to an enzymatic reaction.

It is noted that as the Liberase aqueous solution, a solution obtained by dissolving 5.0 mg of Liberase MNP-S (manufactured by F. Hoffmann-La Roche, Ltd.), which includes an enzyme mixture containing collagenase and neutral protease, in 5.0 mL of water for injection containing human autologous serum of a final concentration of 20% was used.

The enzymatic reaction was carried out at 37°C for 1 hour and 30 minutes.

In addition, in the enzymatic reaction, the ratio of the using amount of the M1 specimen to the using amount of the enzyme is 170 on the mass basis.

The tissue digestion solution containing the synovium-derived mesenchymal stem cells obtained by enzymatically reacting the M1 specimen was transferred to a 50 mL centrifuge tube through a cell strainer, and the centrifugal force was set to 400 g, followed by centrifugation for 5 minutes.

The supernatant was removed, the obtained suspension containing synovium-derived mesenchymal stem cells was suspended in a culture medium, and the entire amount thereof was seeded in a flask ($1.44 \times 10^7$ cells, 1,880 cells/$cm^2$).

In addition, as the culture medium, a culture medium in which human autologous serum was dissolved to a final concentration of 10% by volume in Minimum Essential Medium Eagle Alpha Modification ($\alpha$-MEM) was used.

In addition, culture was carried out in a $CO_2$ incubator (37°C, 5% $CO_2$), and after 2 weeks, synovium-derived mesenchymal stem cells were recovered from the flask.

No culture medium exchange was carried out during the culture period.

As a result of quantifying the amount of the liberase remaining in the culture medium was quantified, it was 59.8 ng/mL.

The number of recovered synovium-derived mesenchymal stem cells was $0.80 \times 10^7$, which reached a level at which an application to a therapeutic agent for a meniscus or a therapeutic agent for osteoarthritis could be examined. In Table 1, this result was evaluated as an evaluated B in the evaluation of the medical treatment applicability.

The ratio (proliferation ratio) of the number of cells recovered after 2 weeks to the number of cells at the time of seeding was 0.6.

It is noted that the number of recovered cells was measured by cell counting with visual observation and by using a dual fluorescence optical cell counter LUNA-FL (registered trade name) (manufactured by Logos Biosystems).

On the 4th day from the culture start date (the seeding), a cell image of a population including synovium-derived mesenchymal stem cells was acquired, and the density information (the estimated density distribution) on the synovium-derived mesenchymal stem cells was derived from cell image according to the kernel density estimation.

Next, a mean-shift was carried out with reference to the estimated density distribution to detect, as the colony, a collection of the mesenchymal stem cells, which had converged to the same maximal point in the estimated density distribution.

As a result of deriving the average colony size and the colony forming unit (CFU) from the detected colonies, the average colony size was 0.743 mm and the CFU was 0.256 CFU/$mm^2$.

It is noted that the acquisition method for the cell image, the derivation method for the estimated density distribution, the detection method for colonies, and the derivation of the average colony size and the CFU were carried out by using the above-described cell image analysis device according to the first embodiment.

In addition, the measurement of the average colony size was carried out with respect to colonies having a colony size of more than 0.54 mm.

For Example 2 below as well, the measurements of the average colony size and the like were carried out according to the same methods.

As a result of examining the expression of cell surface markers peculiar to the recovered synovium-derived mesenchymal stem cells, with an antigen-antibody reaction, the cells were CD90-positive and CD45-negative.

In addition, as a result of subjecting the recovered synovium-derived mesenchymal stem cells to Alcian blue staining, the differentiation potency to cartilage cells was confirmed.

From the above, it was confirmed that the recovered cells are synovium-derived mesenchymal stem cells.

<Example 2>

Synovium-derived mesenchymal stem cells were prepared using a human specimen of a patient with a meniscus injury without sterilizing the synovial tissue.

Specifically, respective tissues of a G0 specimen (synovial tissue: 0.80 g), a G1 specimen (synovial tissue: 0.42 g), a G2 specimen (synovial tissue: 0.60 g), a G3 specimen (synovial tissue: 0.66 g), a G4 specimen (synovial tissue: 0.86 g), and a G5 specimen (synovial tissue: 0.33 g), were shredded with scissors, and each of them was immersed in 5.0 mL of the liberase aqueous solution used in Example 1 and subjected to an enzymatic reaction.

The enzymatic reaction was carried out at 37°C for 3 hours.

In addition, in the enzymatic reaction, the ratios of the using amounts of the G0 specimen, the G1 specimen, the G2 specimen, the G3 specimen, the G4 specimen, and the G5 specimen to the using amount of the enzyme were respectively 160, 84, 120, 132, 172, and 66, on the mass basis.

The tissue digestion solution, which contained the synovium-derived mesenchymal stem cells obtained by enzymatically reacting each of the G0 specimen, the G1 specimen, the G2 specimen, the G3 specimen, the G4 specimen, and the G5 specimen, was transferred to a 50 mL centrifuge tube through a cell strainer, and the centrifugal force was set to 400 g, followed by centrifugation for 5 minutes.

Thereafter, as a washing step, the supernatant was discarded, suspension was carried out again in 20 mL of α-MEM, and the centrifugal force was set to 400 g, followed by centrifugation for 5 minutes. This washing step was repeated twice in total.

The supernatant was removed, each of the obtained suspensions containing synovium-derived mesenchymal stem cells was suspended in a culture medium, and the entire amount thereof was seeded in a flask (for the G0 specimen, $1.43 \times 10^7$ cells and 1,874 cells/cm$^2$), (for the G1 specimen, $0.53 \times 10^7$ cells and 690 cells/cm$^2$), (for the G2 specimen, $0.81 \times 10^7$ cells and 1,059 cells/cm$^2$), (for the G3 specimen, $1.52 \times 10^7$ cells and 1,987 cells/cm$^2$), (for the G4 specimen, $0.79 \times 10^7$ cells and 1,028 cells/cm$^2$), and (for the G5 specimen, $0.60 \times 10^7$ cells and 787 cells/cm$^2$).

It is noted that as the culture medium, α-MEM was used as in Example 1.

In addition, culture was carried out in a $CO_2$ incubator (37°C, 5% $CO_2$), and after 2 weeks, synovium-derived mesenchymal stem cells were recovered from the flask.

No culture medium exchange was carried out during the culture period.

As a result of quantifying the amount of the liberase remaining in the culture medium was quantified, it was 0.1 ng/mL or less for all of the G0 specimen to the G5 specimen (equal to or smaller than the detection limit of the measurement kit).

The number of recovered synovium-derived mesenchymal stem cells was $4.9 \times 10^7$ for the G0 specimen, $4.9 \times 10^7$ for the G1 specimen, $7.6 \times 10^7$ for the G2 specimen, $5.8 \times 10^7$ for the G3 specimen, $7.0 \times 10^7$ for the G4 specimen, and $6.5 \times 10^7$ for the G5 specimen.

The total number of recovered synovium-derived mesenchymal stem cells significantly exceeded $1.0 \times 10^7$, which reached a level at which an amount of cells required for medical treatment could be provided sufficiently and stably.

It is noted that the proliferation ratio was 3.5 for the G0 specimen, 9.5 for the G1 specimen, 9.6 for the G2 specimen, 3.9 for the G3 specimen, 9.1 for the G4 specimen, and 11.0 for the G5 specimen, and thus a remarkable effect of improving the proliferation ratio was observed. In Table 1, this result was evaluated as an evaluated A in the evaluation of the medical treatment applicability.

On the 4th day to 6th day from the culture start date (the seeding), a cell image of a population including synovium-derived mesenchymal stem cells was acquired, and the density information (the estimated density distribution) on the synovium-derived mesenchymal stem cells was derived from the cell image according to the kernel density estimation.

Next, a mean-shift was carried out with reference to the estimated density distribution to detect, as the colony, a collection of the mesenchymal stem cells, which had converged to the same maximal point in the estimated density distribution.

As a result of deriving the average colony size and the colony forming unit (CFU) from the detected colonies, they were as follows.

- · G0 specimen: colonies were detected 6 days after the culture start date, average colony size: 1.150 mm, CFU: 0.947/mm$^2$
- · G1 specimen: colonies were detected 5 days after the culture start date, average colony size: 0.830 mm, CFU: 0.344/mm$^2$
- · G2 specimen: colonies were detected 4 days after the culture start date, average colony size: 0.782 mm, CFU: 0.368 / mm $^2$

- G3 specimen: colonies were detected 4 days after the culture start date, average colony size: 0.816 mm, CFU: 0.311/mm$^2$
- G4 specimen: colonies were detected 6 days after the culture start date, average colony size: 1.030 mm, CFU: 0.575/mm$^2$
- G5 specimen: colonies were detected 4 days after the culture start date, average colony size: 0.777 mm, CFU: 0.340/mm$^2$

As a result of examining the expression of cell surface markers peculiar to the recovered synovium-derived mesenchymal stem cells, with an antigen-antibody reaction, the cells were CD90-positive and CD45-negative.
In addition, as a result of subjecting the recovered synovium-derived mesenchymal stem cells to Alcian blue staining, the differentiation potency to cartilage cells was confirmed.
From the above, it was confirmed that the recovered cells are synovium-derived mesenchymal stem cells.

[Table 1]

| | Example 1 | Example 2 | | | | | |
|---|---|---|---|---|---|---|---|
| Specimen | M1 | G0 | G1 | G2 | G3 | G4 | G5 |
| Tissue weight (g) | 0.85 | 0.80 | 0.42 | 0.60 | 0.66 | 0.86 | 0.33 |
| Number of seeded cells ($\times 10^7$ cells) | 1.44 | 1.43 | 0.53 | 0.81 | 1.52 | 0.79 | 0.60 |
| Number of recovered cells ($\times 10^7$ cells) | 0.80 | 4.90 | 4.90 | 7.60 | 5.80 | 7.00 | 6.50 |
| Proliferation ratio | 0.6 | 3.5 | 9.5 | 9.6 | 3.9 | 9.1 | 11 |
| Average colony size (mm) | 0.743 | 1.150 | 0.839 | 0.782 | 0.816 | 1.030 | 0.777 |
| CFU (/mm$^2$) | 0.256 | 0.947 | 0.344 | 0.368 | 0.311 | 0.574 | 0.340 |
| Evaluation of medical treatment applicability | B | A | A | A | A | A | A |

From the results shown in Table 1, it can see that the therapeutic agent for arthrosis, which is produced by the production method for a therapeutic agent for arthrosis, makes it possible to carry out a proliferation prediction for mesenchymal stem cells in the middle of culture with excellent accuracy and makes it possible to produce a therapeutic agent for arthrosis suitable for the treatment of arthrosis.
In addition, it can be seen that the average colony size and the CFU of mesenchymal stem cells during culture contribute to the number and the proliferation ratio of the recovered cells.

«Application as therapeutic agent for meniscus»

The synovium-derived mesenchymal stem cells produced from the G0 specimens to the G5 specimens in Example 2 were transplanted to a meniscus injury site of a patient with a meniscus injury as an autologous cell therapy.
In the patient with a meniscus injury transplanted with the synovium-derived mesenchymal stem cells, favorable symptom alleviation was observed by 52 weeks after the transplantation.
An improvement of 22 points or more was confirmed in terms of the Lysholm score (which was obtained by an evaluation staff, see Table 2 below), which is known as a symptom score, until 52 weeks after the transplantation compared with that before the medical treatment, and the reposition was also confirmed in the meniscus checking by magnetic resonance imaging (MRI), which indicated the medical treatment results were favorable.

[Table 2]

| Evaluation item | | Score |
|---|---|---|
| Limping (foot limping) (5 points) | Absent | 5 |
| | Seldom or often | 3 |
| | Always severe | 0 |
| Walking unstability (30 points) | Walking is possible without problem | 30 |
| | Knees rarely collapse during sports or other vigorous exercise | 25 |
| | Knees frequently collapse during sports or other vigorous exercise | 15 |
| | Unstable at times in everyday life | 10 |
| | Unstable frequently in everyday life | 5 |
| | Always unstable when walking | 0 |
| Supporting ability (5 points) | Fully loadable | 5 |
| | Stick or crutch is necessary | 3 |
| | Loading application is impossible | 0 |
| Swelling (10 points) | Absent | 10 |
| | Associated with knee collapse | 7 |
| | Due to vigorous exercise | 5 |
| | With exercise in everyday life | 2 |
| | Always | 0 |
| Going up and down stairs (5 points) | No problem | 5 |
| | Some problems | 3 |
| | Going up and down is possible only step by step | 2 |
| | Impossible | 0 |
| Pain (30 points) | Absent | 30 |
| | Occasionally slight during vigorous exercise | 25 |
| | At time when knees collapse | 20 |
| | During vigorous exercise | 15 |
| | During or after walking 2 km or more | 10 |
| | During or after walking less than 2 km | 5 |
| | Always | 0 |
| Bending and stretching of knee (5 points) | No problem | 5 |
| | Some problems | 3 |
| | Up to 90 degrees | 2 |
| | Impossible | 0 |
| Femur atrophy (5 points) | Absent | 5 |
| | Atrophy of 1 to 2 cm | 3 |
| | Atrophy of 2 cm | 0 |

## Claims

1. A production method for a therapeutic agent for arthrosis, comprising:

   imaging a population including a plurality of mesenchymal stem cells being cultured, to acquire a cell image;
   analyzing the cell image to derive density information on the mesenchymal stem cells in the cell image;
   detecting colonies, which are formed by the mesenchymal stem cells in the population, based on the density information; and
   sorting the population based on at least one of an average colony size or a colony forming unit, which is derived from the detected colonies.

2. The production method for a therapeutic agent for arthrosis according to claim 1, wherein the density information is an estimated density distribution of the mesenchymal stem cells in the cell image, which is derived from kernel density estimation.

3. The production method for a therapeutic agent for arthrosis according to claim 2, wherein the detection of the colonies is carried out by carrying out a mean-shift with reference to the estimated density distribution and detecting, as the colony, a collection of the mesenchymal stem cells, which converges to the same maximal point in the estimated density distribution.

4. The production method for a therapeutic agent for arthrosis according to any one of claims 1 to 3,

   wherein the detection of the colonies is carried out on a 4th day to a 6th day from a culture start date, and
   the sorting of the population is carried out by sorting a population that satisfies at least one of the following condition a1) or b1),
   a1) the average colony size of the mesenchymal stem cells included in the population at any time point of the 4th day to the 6th day from the culture start date start date is 0.740 mm or more, or
   b 1) the colony forming unit of the mesenchymal stem cells included in the population at any time point of the 4th day to the 6th day from the culture start date is 0.250 units/$mm^2$ or more.

5. The production method for a therapeutic agent for arthrosis according to any one of claims 1 to 4, wherein the mesenchymal stem cells are obtained by treating a biological tissue with an enzyme.

6. The production method for a therapeutic agent for arthrosis according to claim 5, wherein in the treatment of the biological tissue with an enzyme, an enzyme mixture containing at least one kind of collagenase and at least one kind of neutral protease is used.

7. The production method for a therapeutic agent for arthrosis according to claim 5 or 6, wherein in the treatment of the biological tissue with an enzyme, a ratio of a using amount of the biological tissue to a using amount of the enzyme is 10 to 1,000 on a mass basis.

8. The production method for a therapeutic agent for arthrosis according to any one of claims 5 to 7, wherein the mesenchymal stem cells are obtained by washing a tissue digestion solution which is obtained by the treatment of the biological tissue with an enzyme until a residual enzyme concentration in a supernatant is 0.5 ng/mL or less.

9. The production method for a therapeutic agent for arthrosis according to any one of claims 1 to 8, wherein the mesenchymal stem cells are a synovium-derived cell.

10. The production method for a therapeutic agent for arthrosis according to any one of claims 1 to 9, wherein the mesenchymal stem cells are human-derived cells.

11. A therapeutic agent for arthritis, which is produced by the production method for a therapeutic agent for arthrosis according to any one of claims 1 to 10.

FIG. 1

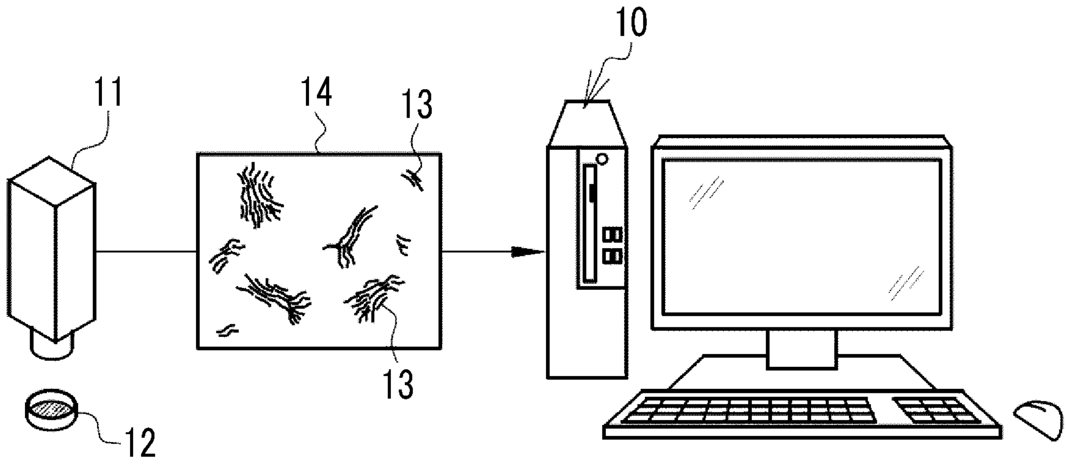
10
11
14
13
13
12

FIG. 2

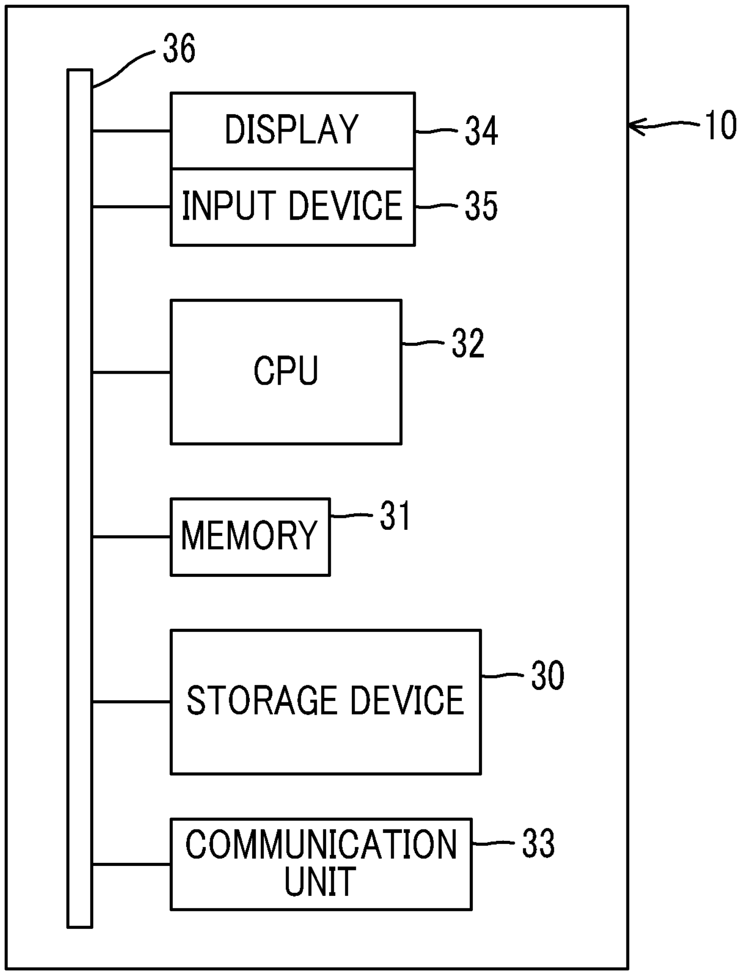
36
DISPLAY
34
INPUT DEVICE
35
10
CPU
32
MEMORY
31
STORAGE DEVICE
30
COMMUNICATION UNIT
33

FIG. 3

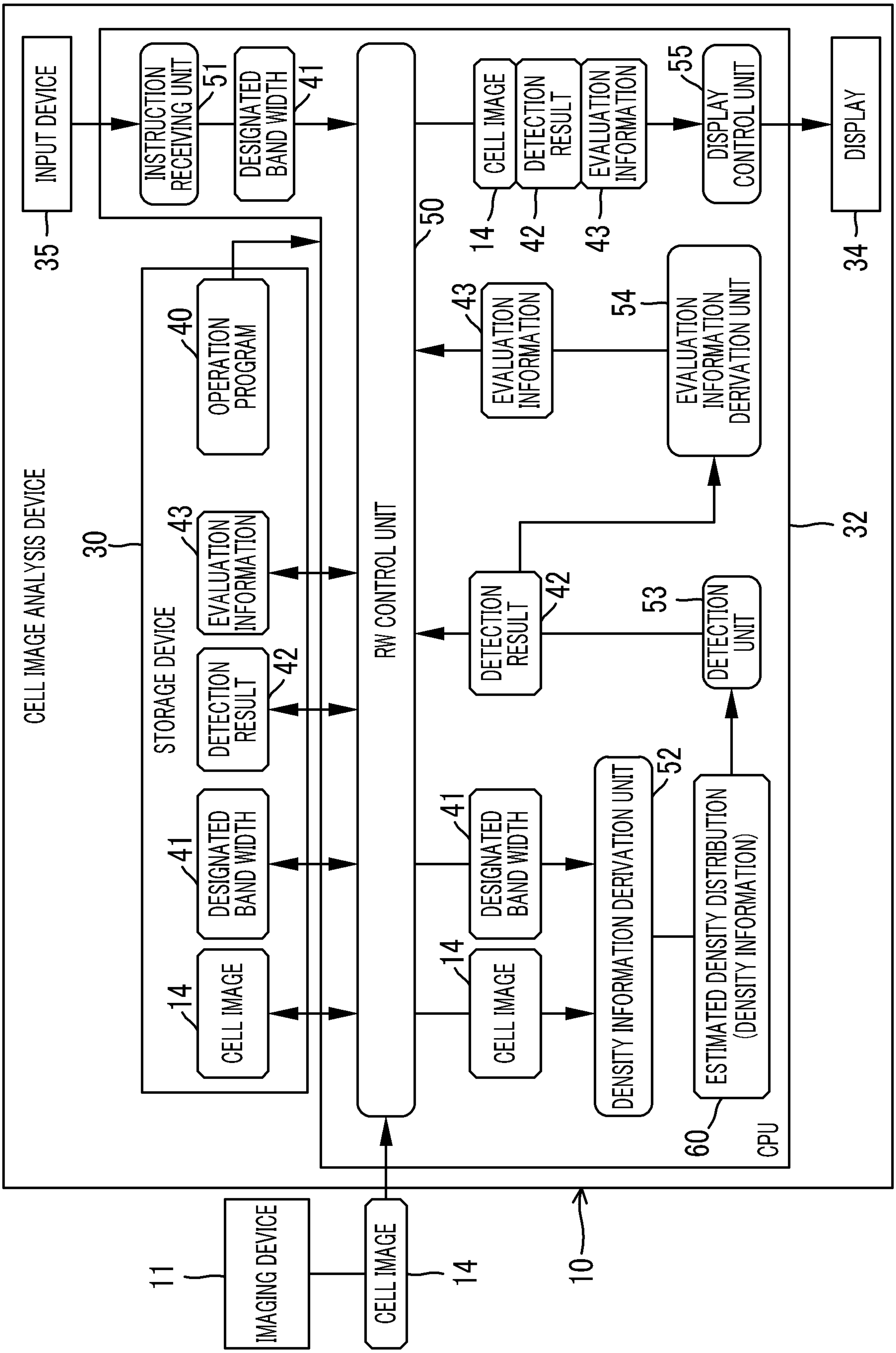
CELL IMAGE ANALYSIS DEVICE
INPUT DEVICE
35
INSTRUCTION RECEIVING UNIT
51
DESIGNATED BAND WIDTH
41
RW CONTROL UNIT
50
CELL IMAGE
14
DETECTION RESULT
42
EVALUATION INFORMATION
43
DISPLAY CONTROL UNIT
55
DISPLAY
34
STORAGE DEVICE
30
OPERATION PROGRAM
40
EVALUATION INFORMATION DERIVATION UNIT
54
DETECTION UNIT
53
DENSITY INFORMATION DERIVATION UNIT
52
ESTIMATED DENSITY DISTRIBUTION (DENSITY INFORMATION)
60
CPU
32
IMAGING DEVICE
11
10

FIG. 4

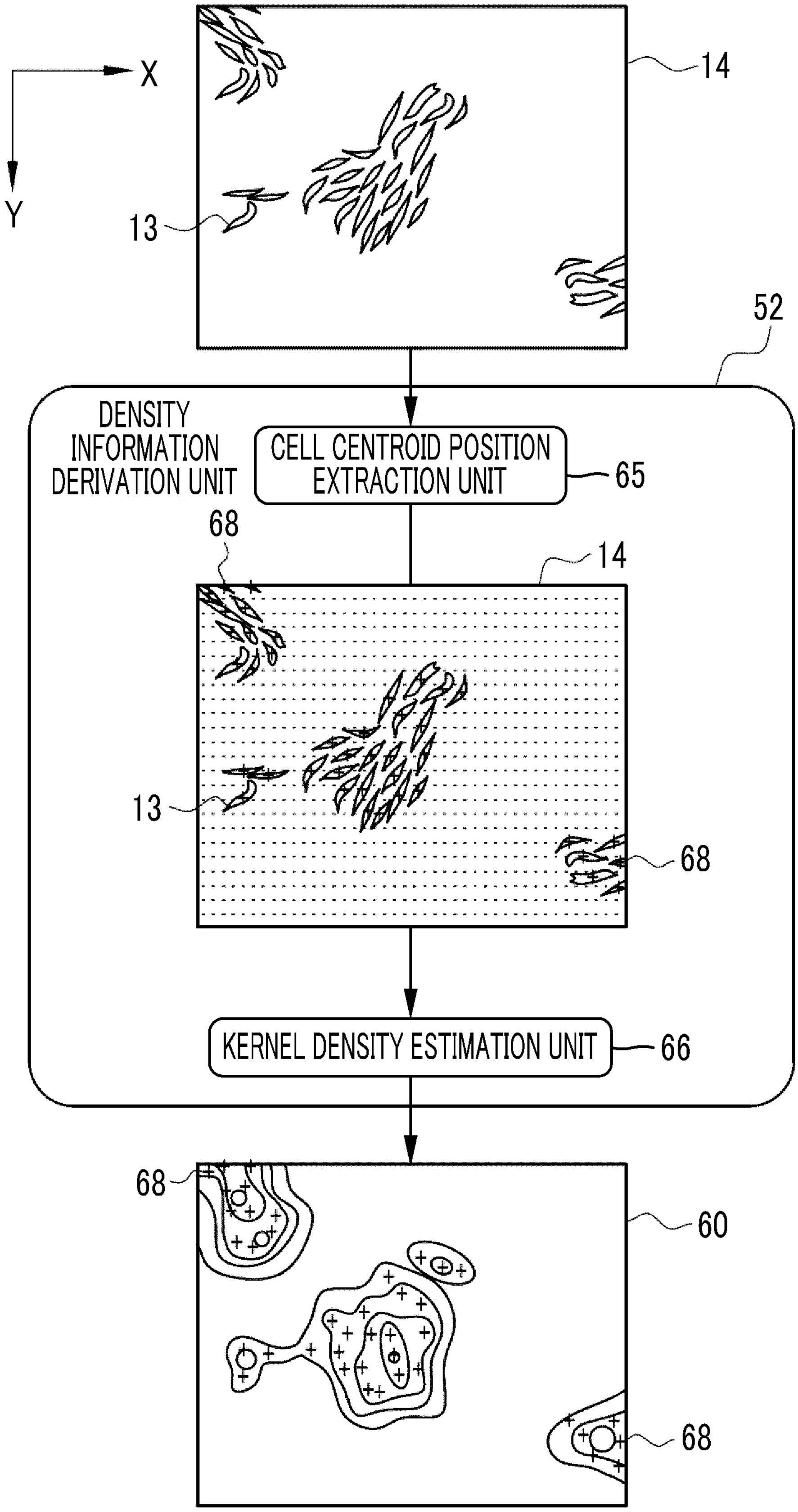
X
Y
14
13
52
DENSITY
INFORMATION
DERIVATION UNIT
CELL CENTROID POSITION
EXTRACTION UNIT
65
68
14
13
68
KERNEL DENSITY ESTIMATION UNIT
66
68
60
68

FIG. 5

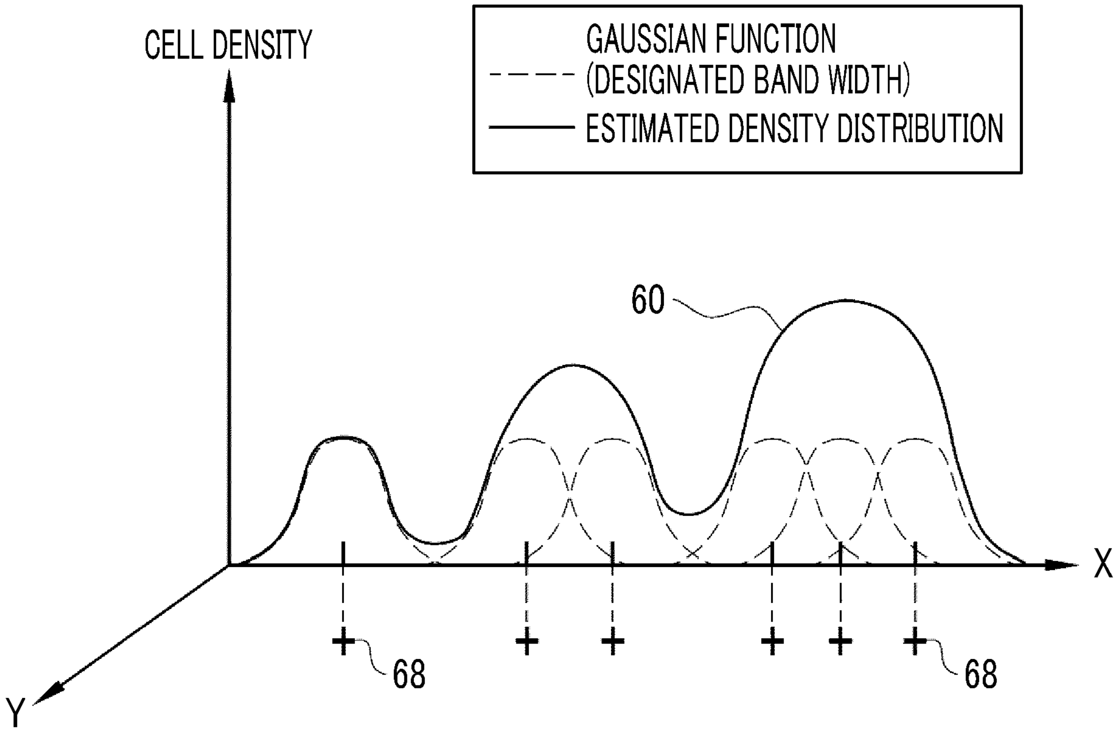
CELL DENSITY
GAUSSIAN FUNCTION
(DESIGNATED BAND WIDTH)
ESTIMATED DENSITY DISTRIBUTION
60
68
68
X
Y

FIG. 6

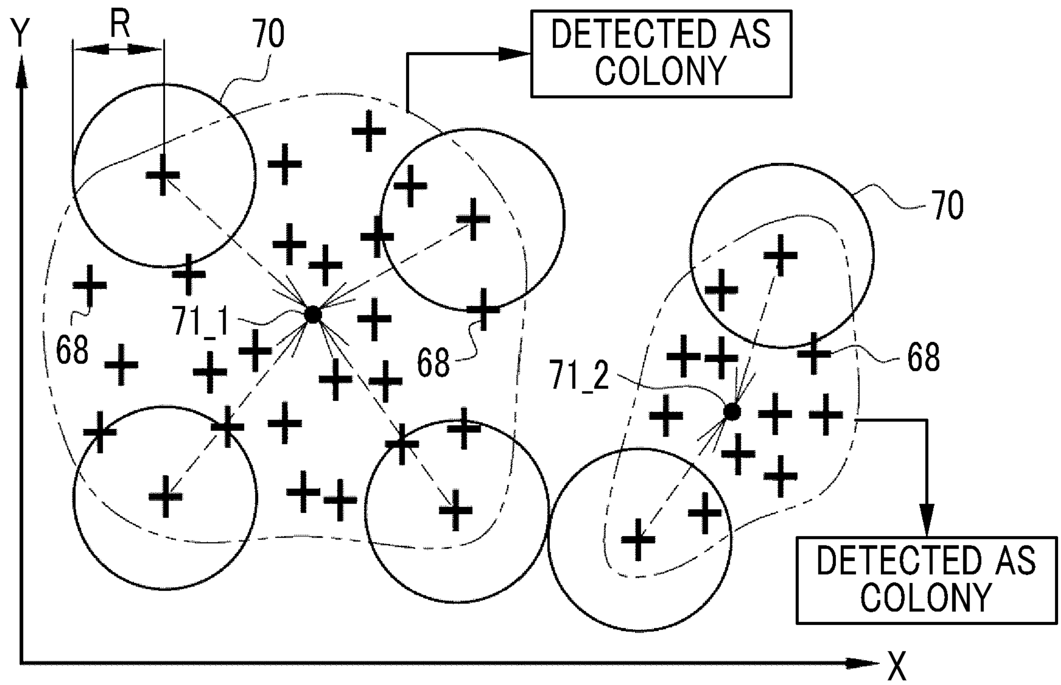
Y
R
70
DETECTED AS COLONY
70
71_1
68
68
71_2
68
DETECTED AS COLONY
X

FIG. 7

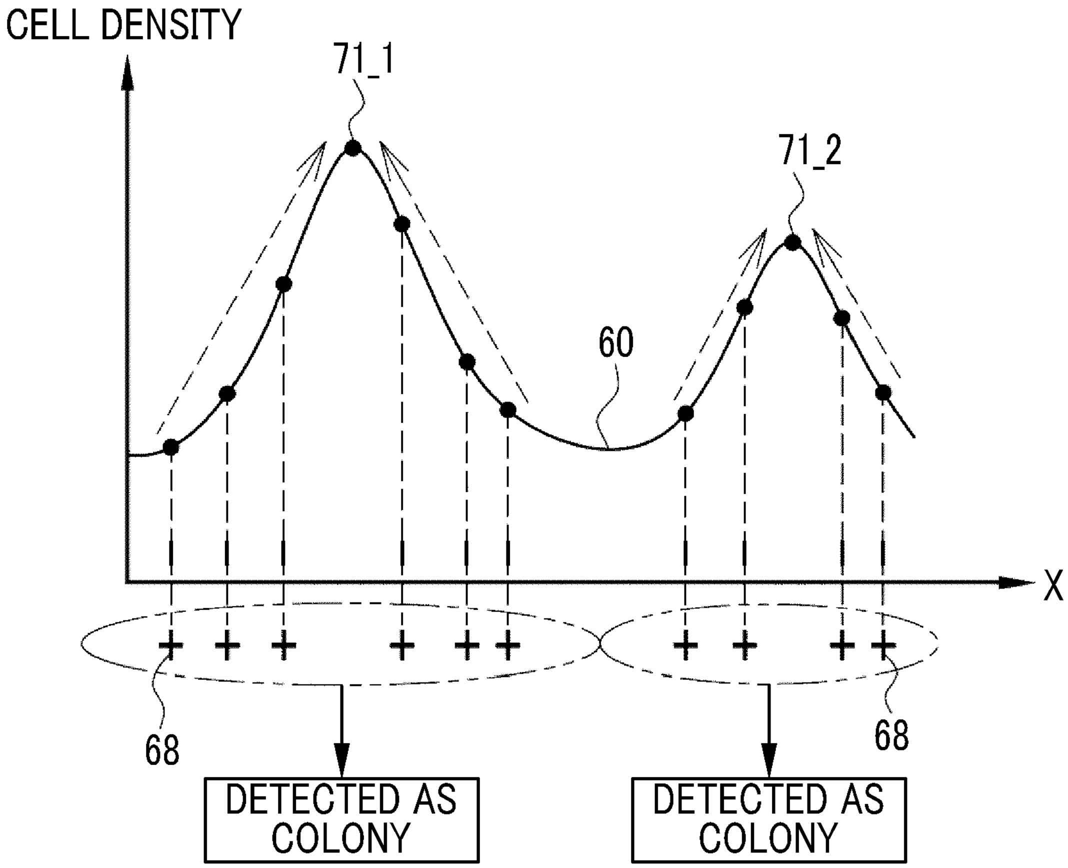
CELL DENSITY
71_1
71_2
60
X
68
68
DETECTED AS COLONY
DETECTED AS COLONY

FIG. 8

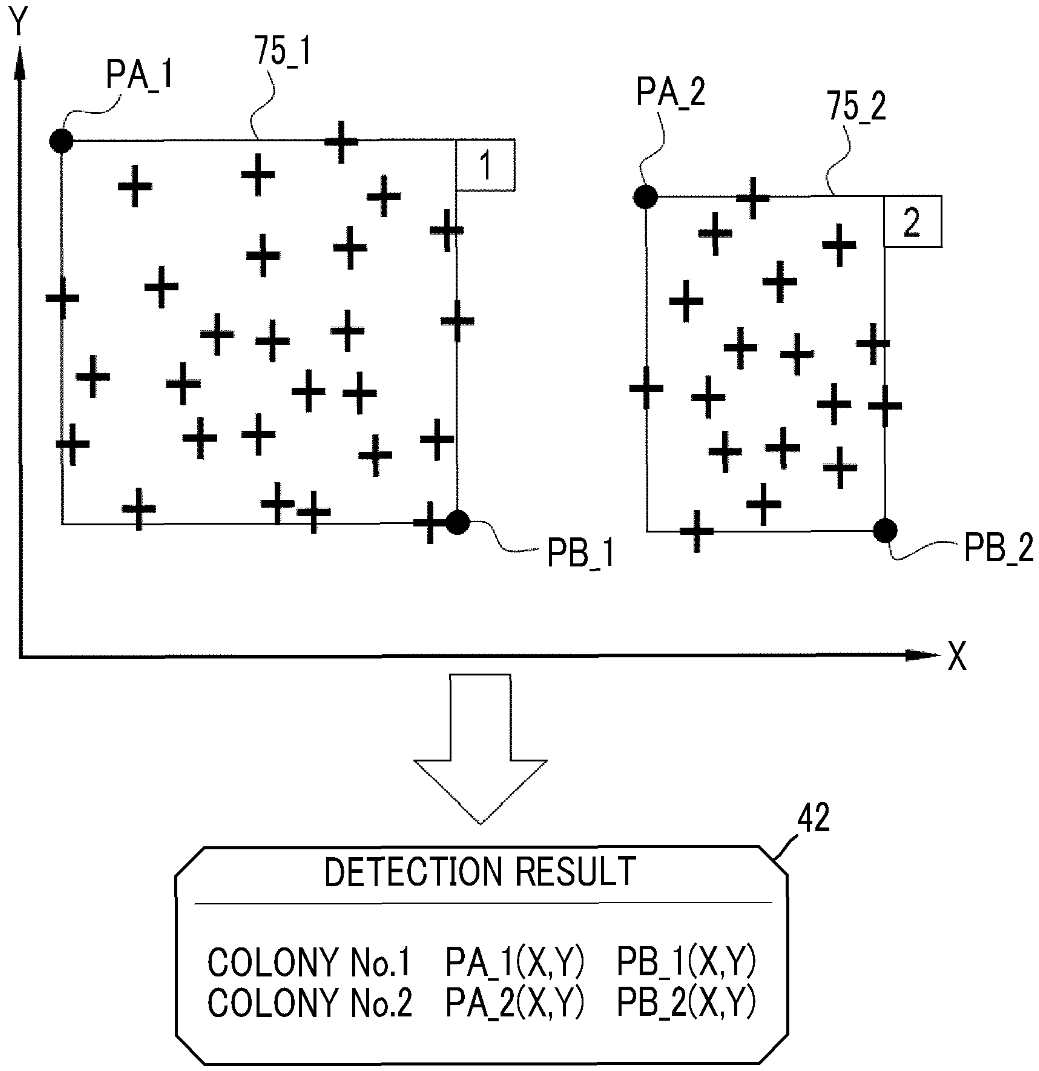
Y
PA_1
75_1
1
PA_2
75_2
2
PB_1
PB_2
X
42
DETECTION RESULT
COLONY No.1 PA_1(X,Y) PB_1(X,Y)
COLONY No.2 PA_2(X,Y) PB_2(X,Y)

FIG. 9

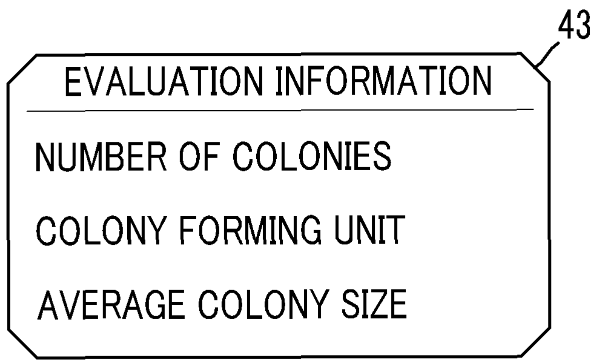
43
EVALUATION INFORMATION
NUMBER OF COLONIES
COLONY FORMING UNIT
AVERAGE COLONY SIZE

FIG. 10

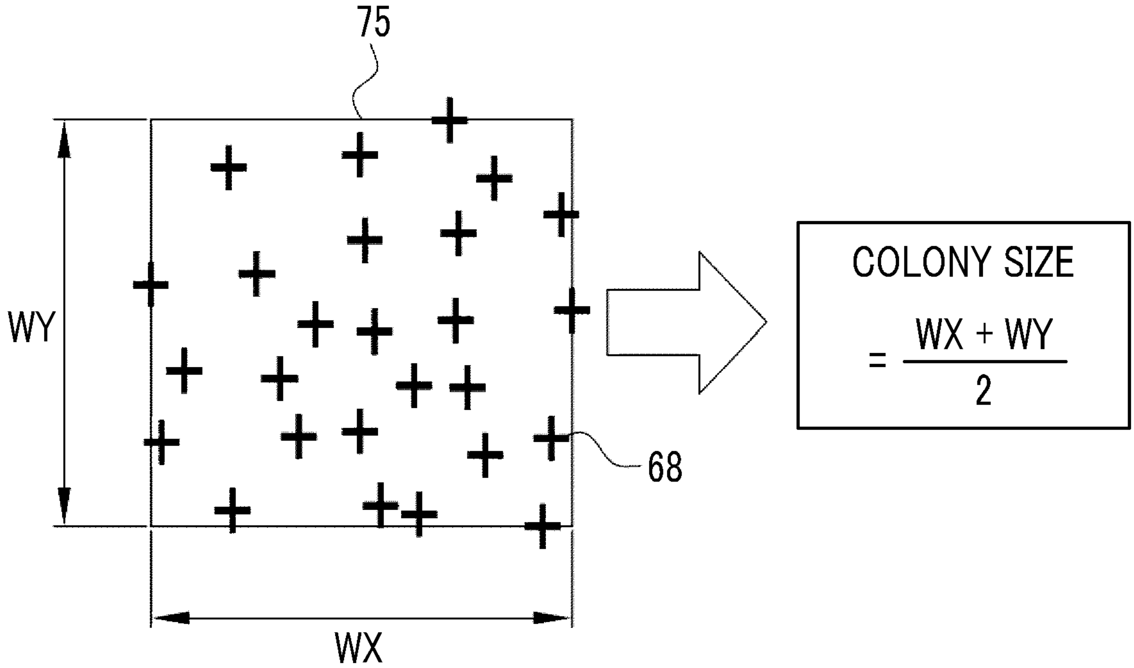

FIG. 11

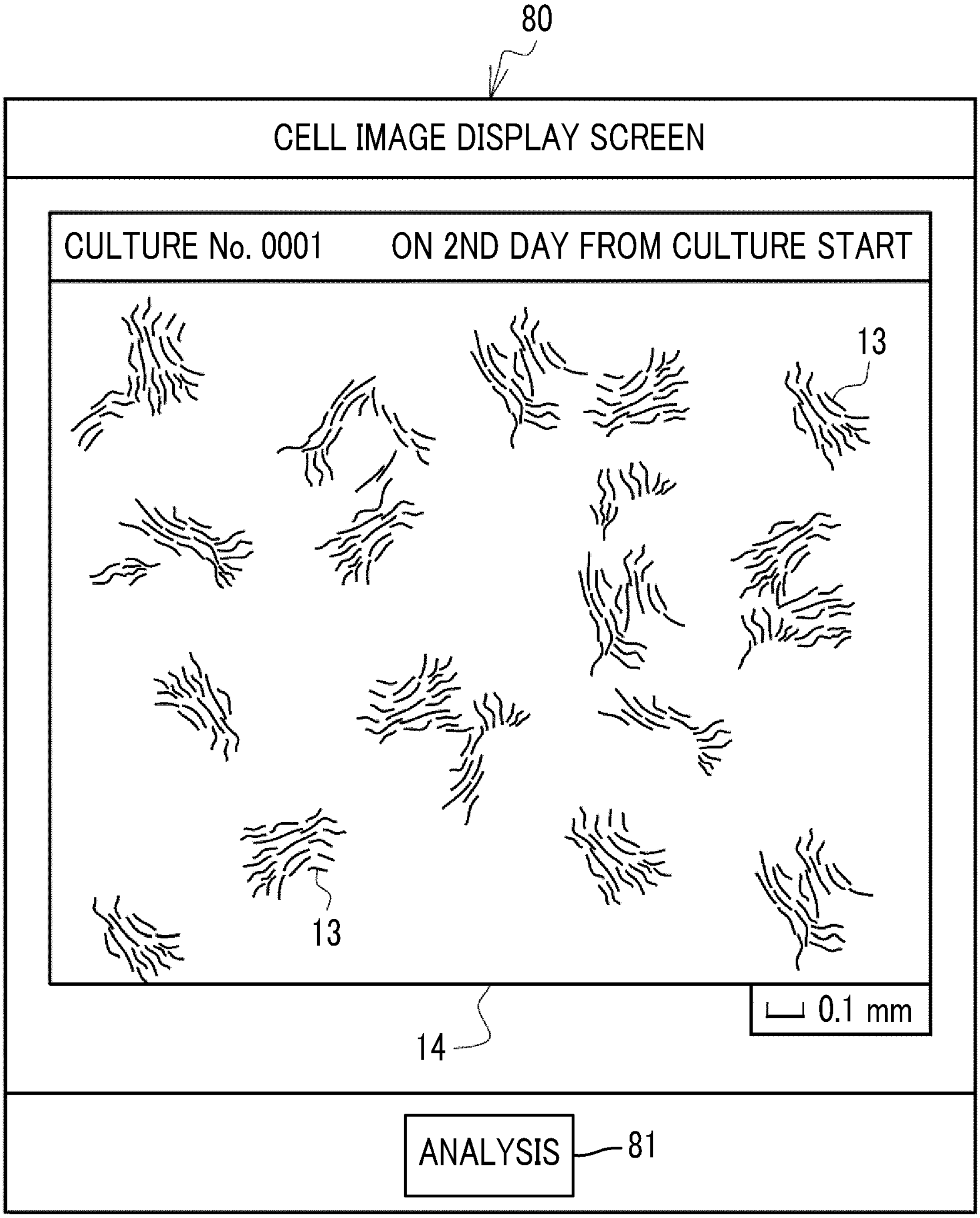
80
CELL IMAGE DISPLAY SCREEN
CULTURE No. 0001
ON 2ND DAY FROM CULTURE START
13
13
14
0.1 mm
ANALYSIS
81

FIG. 12

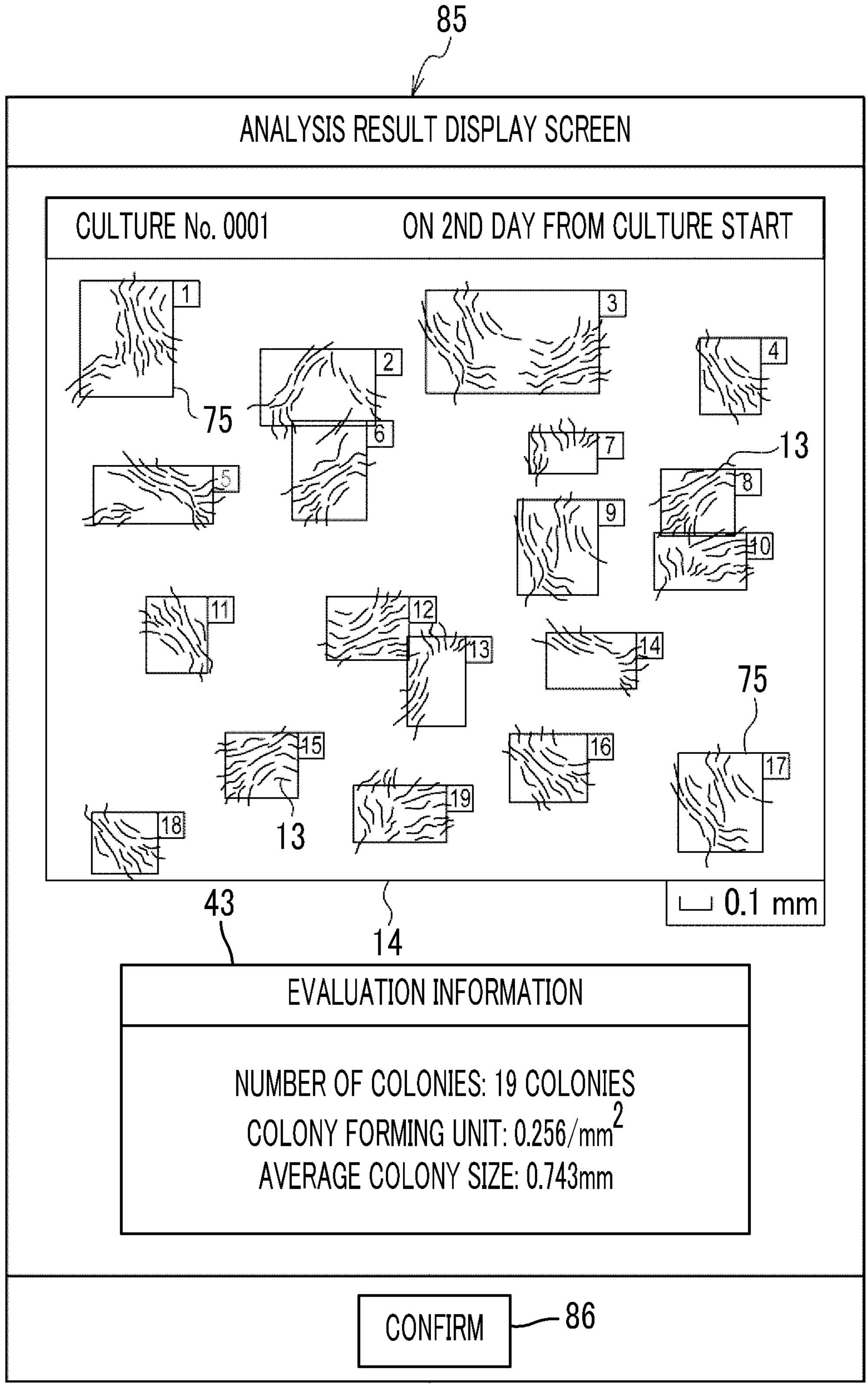
85
ANALYSIS RESULT DISPLAY SCREEN
CULTURE No. 0001
ON 2ND DAY FROM CULTURE START
1
2
3
4
5
6
7
8
9
10
11
12
13
14
15
16
17
18
19
75
13
75
13
0.1 mm
43
14
EVALUATION INFORMATION
NUMBER OF COLONIES: 19 COLONIES
COLONY FORMING UNIT: 0.256/mm2
AVERAGE COLONY SIZE: 0.743mm
CONFIRM
86

FIG. 13

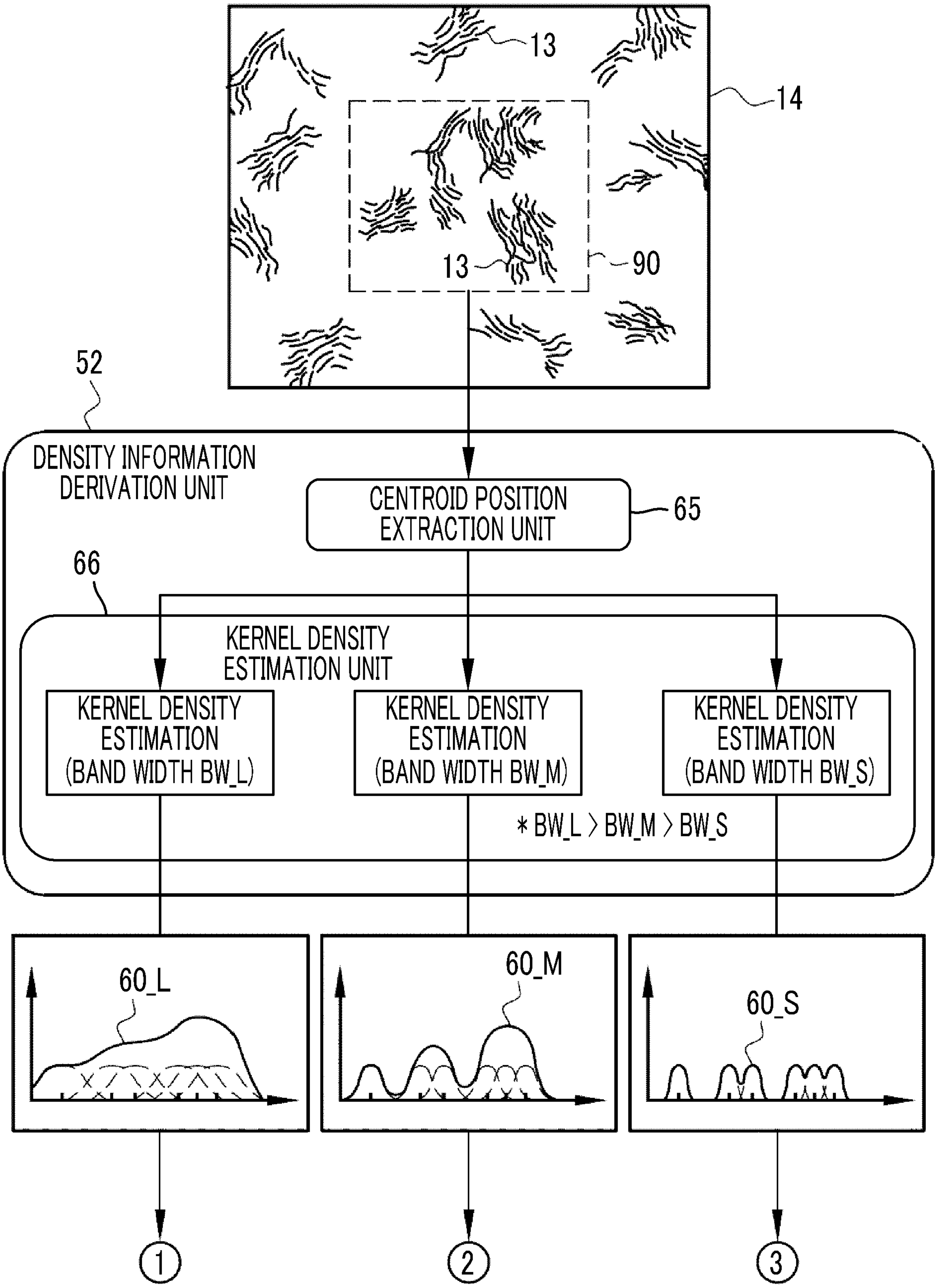

13
14
13
90
52
DENSITY INFORMATION DERIVATION UNIT
CENTROID POSITION EXTRACTION UNIT
65
66
KERNEL DENSITY ESTIMATION UNIT
KERNEL DENSITY ESTIMATION (BAND WIDTH BW_L)
KERNEL DENSITY ESTIMATION (BAND WIDTH BW_M)
KERNEL DENSITY ESTIMATION (BAND WIDTH BW_S)
* BW_L > BW_M > BW_S
60_L
60_M
60_S
①
②
③

FIG. 14

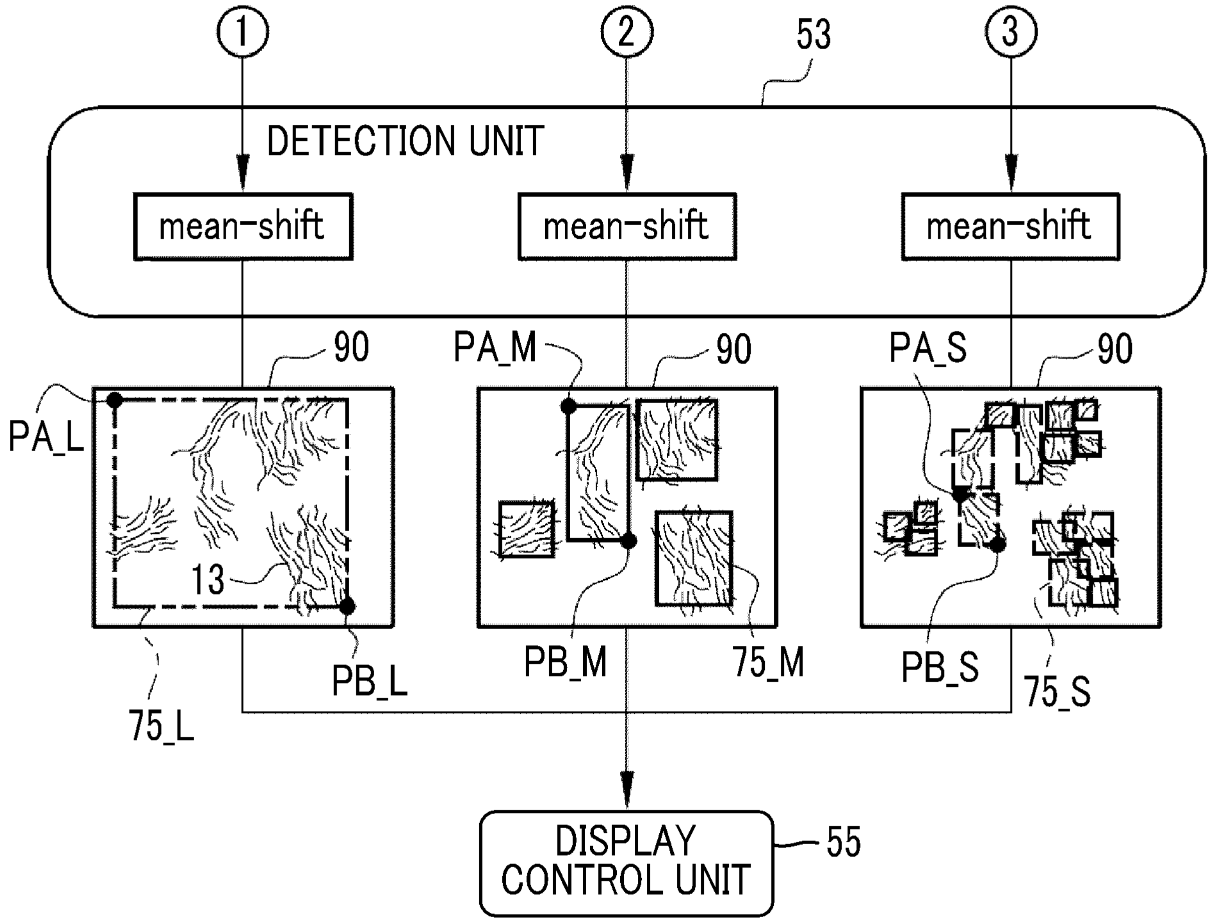

1
2
3
53
DETECTION UNIT
mean-shift
mean-shift
mean-shift
90
PA_M
90
PA_S
90
PA_L
13
PB_L
75_L
PB_M
75_M
PB_S
75_S
DISPLAY CONTROL UNIT
55

FIG. 15

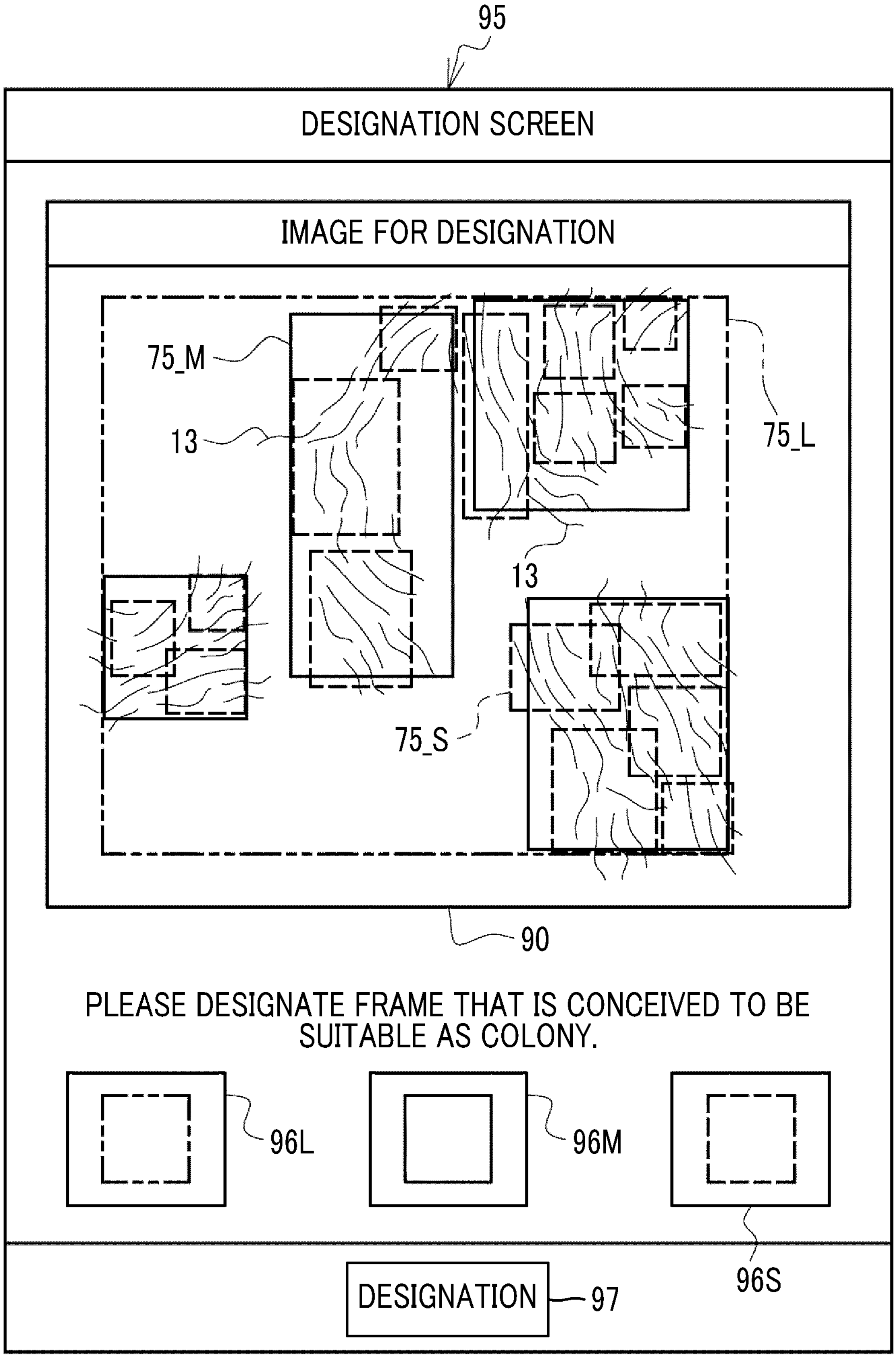
95
DESIGNATION SCREEN
IMAGE FOR DESIGNATION
75_M
13
75_L
13
75_S
90
PLEASE DESIGNATE FRAME THAT IS CONCEIVED TO BE SUITABLE AS COLONY.
96L
96M
96S
DESIGNATION
97

FIG. 16

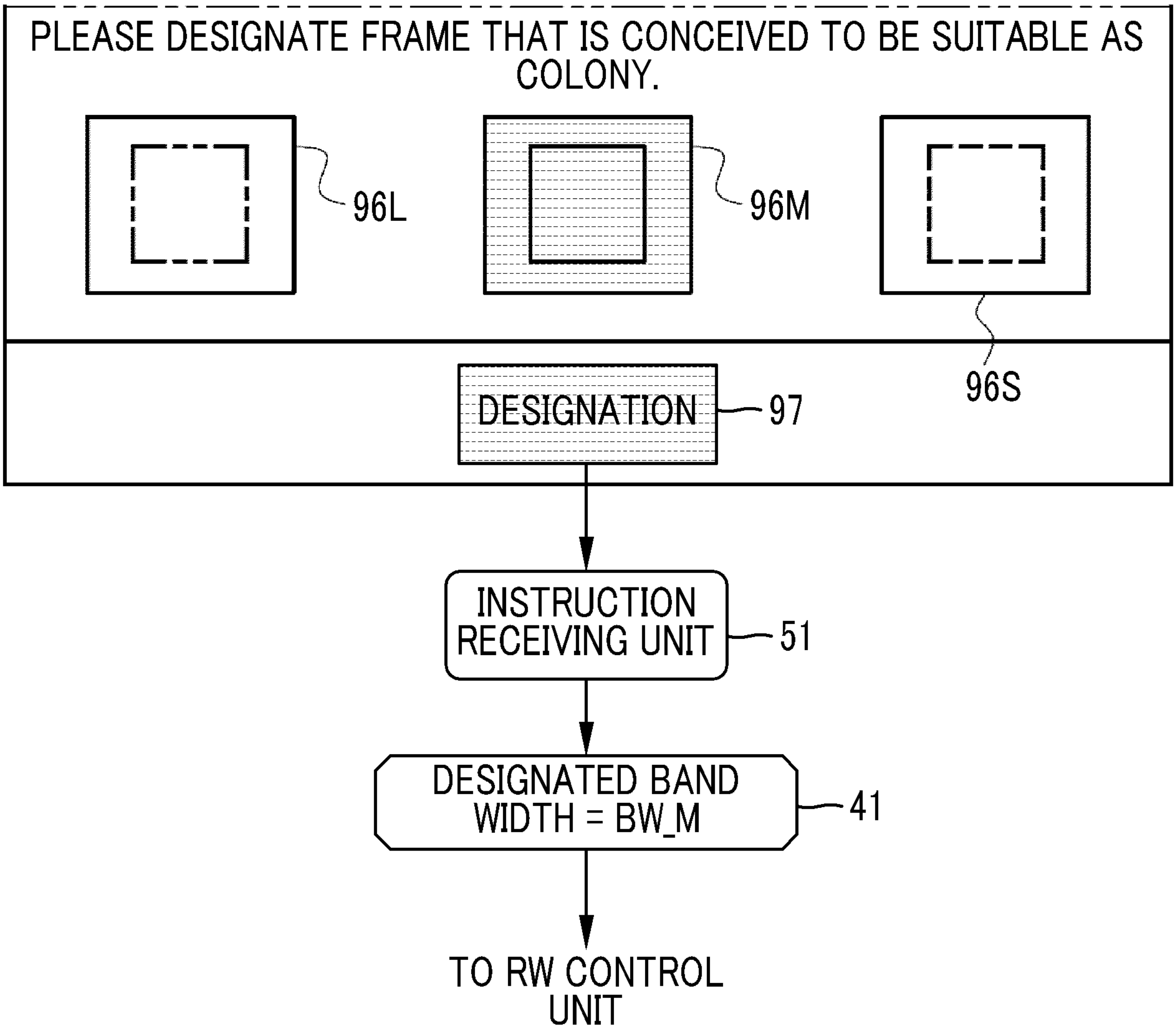
PLEASE DESIGNATE FRAME THAT IS CONCEIVED TO BE SUITABLE AS COLONY.
96L
96M
96S
DESIGNATION
97
INSTRUCTION RECEIVING UNIT
51
DESIGNATED BAND WIDTH = BW_M
41
TO RW CONTROL UNIT

FIG. 17

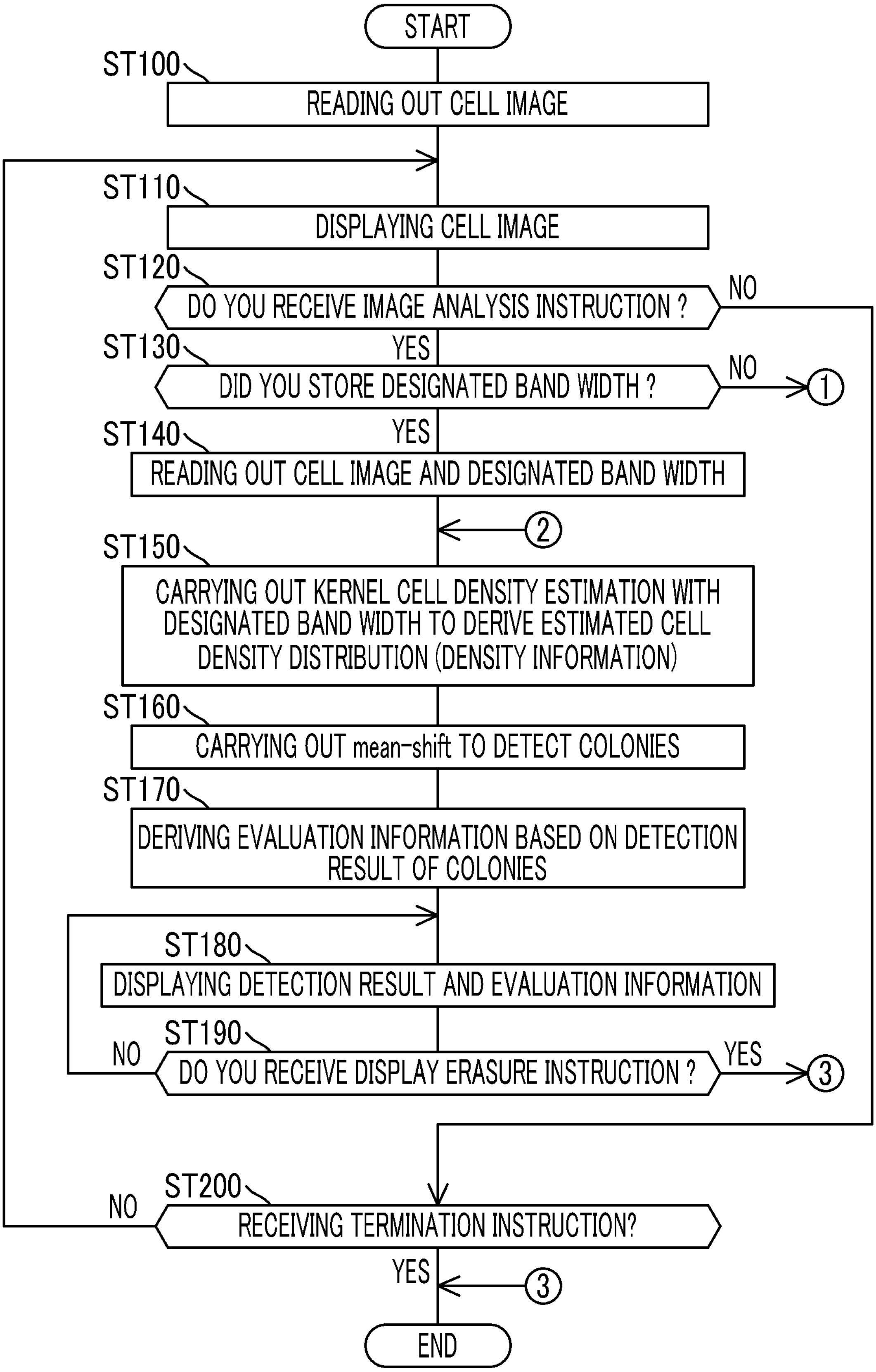
START
ST100
READING OUT CELL IMAGE
ST110
DISPLAYING CELL IMAGE
ST120
DO YOU RECEIVE IMAGE ANALYSIS INSTRUCTION ?
NO
YES
ST130
DID YOU STORE DESIGNATED BAND WIDTH ?
NO
1
YES
ST140
READING OUT CELL IMAGE AND DESIGNATED BAND WIDTH
2
ST150
CARRYING OUT KERNEL CELL DENSITY ESTIMATION WITH DESIGNATED BAND WIDTH TO DERIVE ESTIMATED CELL DENSITY DISTRIBUTION (DENSITY INFORMATION)
ST160
CARRYING OUT mean-shift TO DETECT COLONIES
ST170
DERIVING EVALUATION INFORMATION BASED ON DETECTION RESULT OF COLONIES
ST180
DISPLAYING DETECTION RESULT AND EVALUATION INFORMATION
ST190
NO
DO YOU RECEIVE DISPLAY ERASURE INSTRUCTION ?
YES
3
ST200
NO
RECEIVING TERMINATION INSTRUCTION?
YES
3
END

FIG. 18

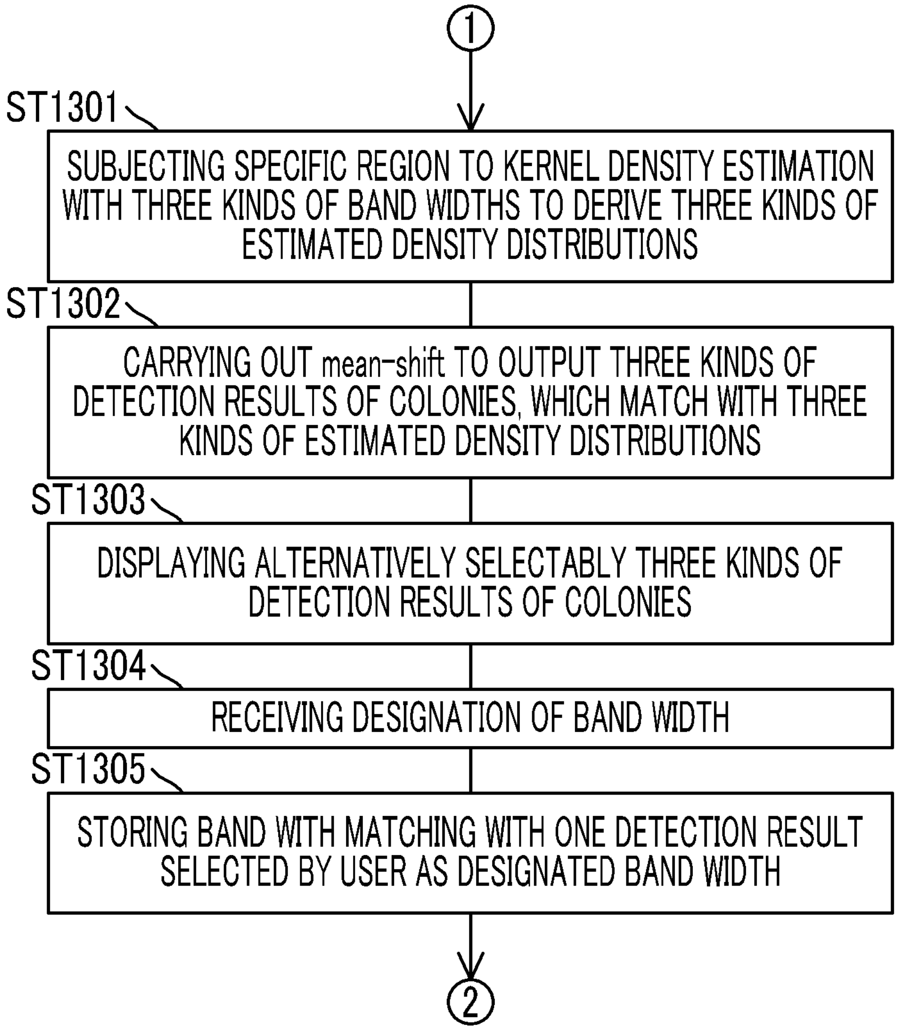
1
ST1301
SUBJECTING SPECIFIC REGION TO KERNEL DENSITY ESTIMATION WITH THREE KINDS OF BAND WIDTHS TO DERIVE THREE KINDS OF ESTIMATED DENSITY DISTRIBUTIONS
ST1302
CARRYING OUT mean-shift TO OUTPUT THREE KINDS OF DETECTION RESULTS OF COLONIES, WHICH MATCH WITH THREE KINDS OF ESTIMATED DENSITY DISTRIBUTIONS
ST1303
DISPLAYING ALTERNATIVELY SELECTABLY THREE KINDS OF DETECTION RESULTS OF COLONIES
ST1304
RECEIVING DESIGNATION OF BAND WIDTH
ST1305
STORING BAND WITH MATCHING WITH ONE DETECTION RESULT SELECTED BY USER AS DESIGNATED BAND WIDTH
2

FIG. 19

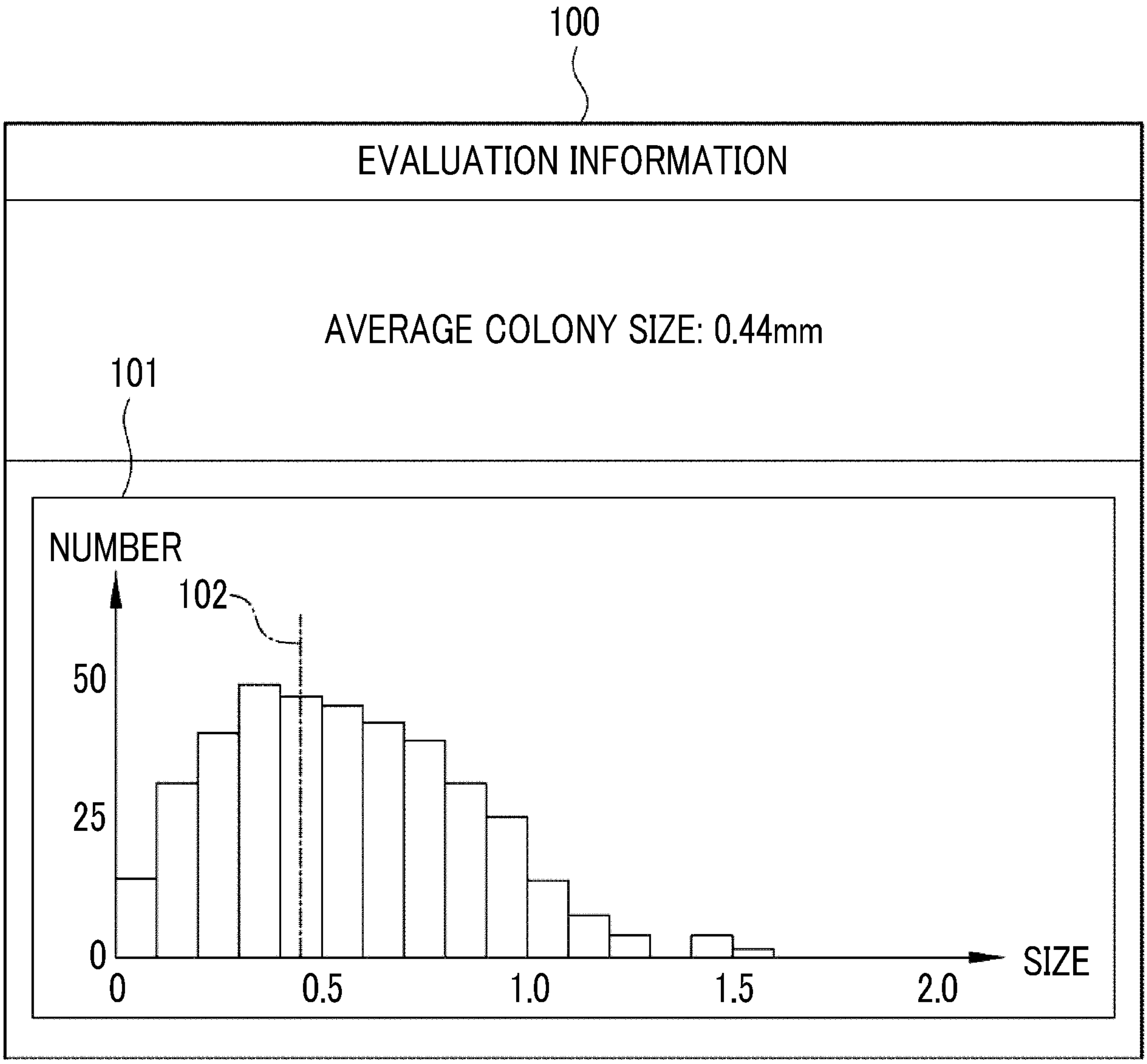
100
EVALUATION INFORMATION
AVERAGE COLONY SIZE: 0.44mm
101
NUMBER
102
50
25
0
0
0.5
1.0
1.5
2.0
SIZE

FIG. 20

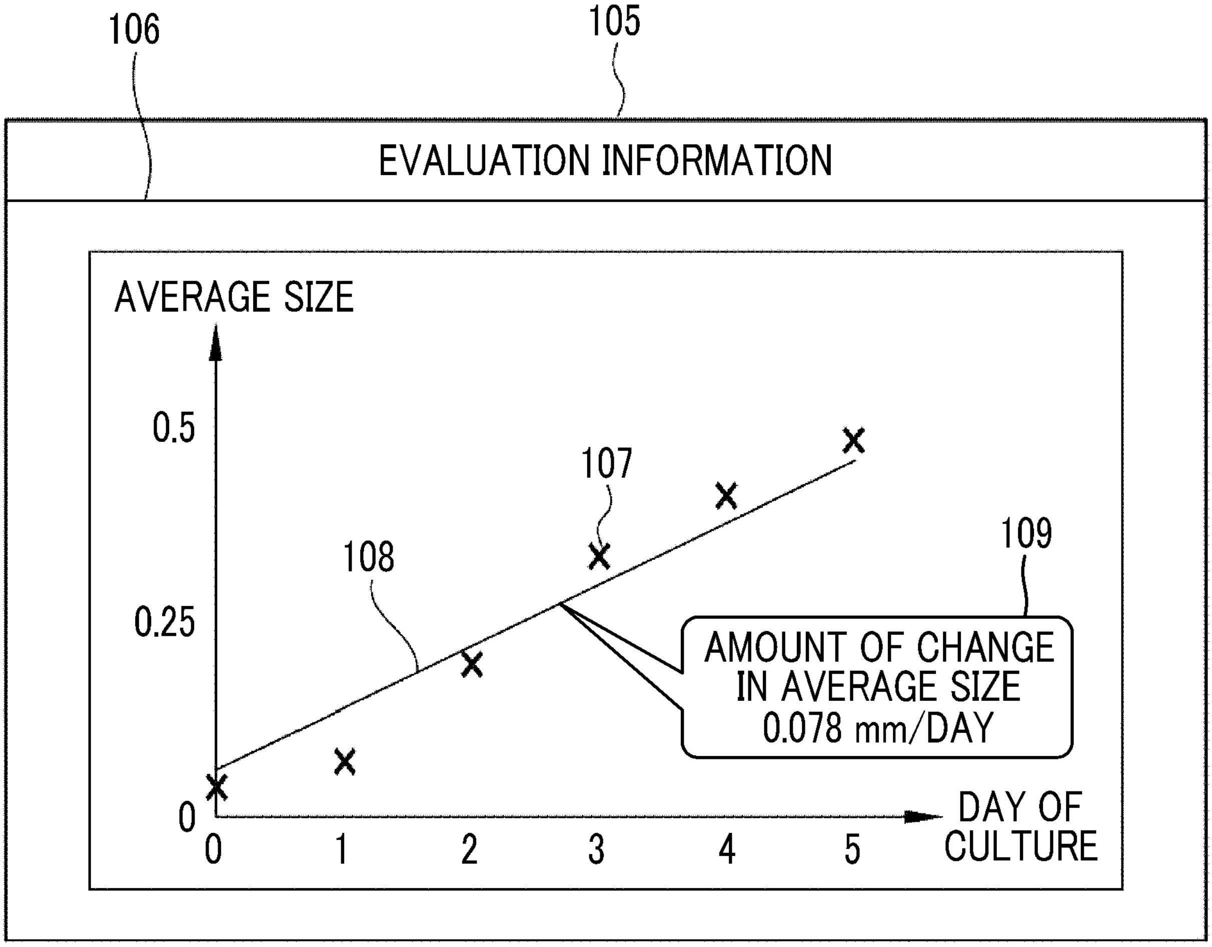
106
105
EVALUATION INFORMATION
AVERAGE SIZE
0.5
0.25
0
107
108
109
AMOUNT OF CHANGE IN AVERAGE SIZE 0.078 mm/DAY
0
1
2
3
4
5
DAY OF CULTURE

FIG. 21

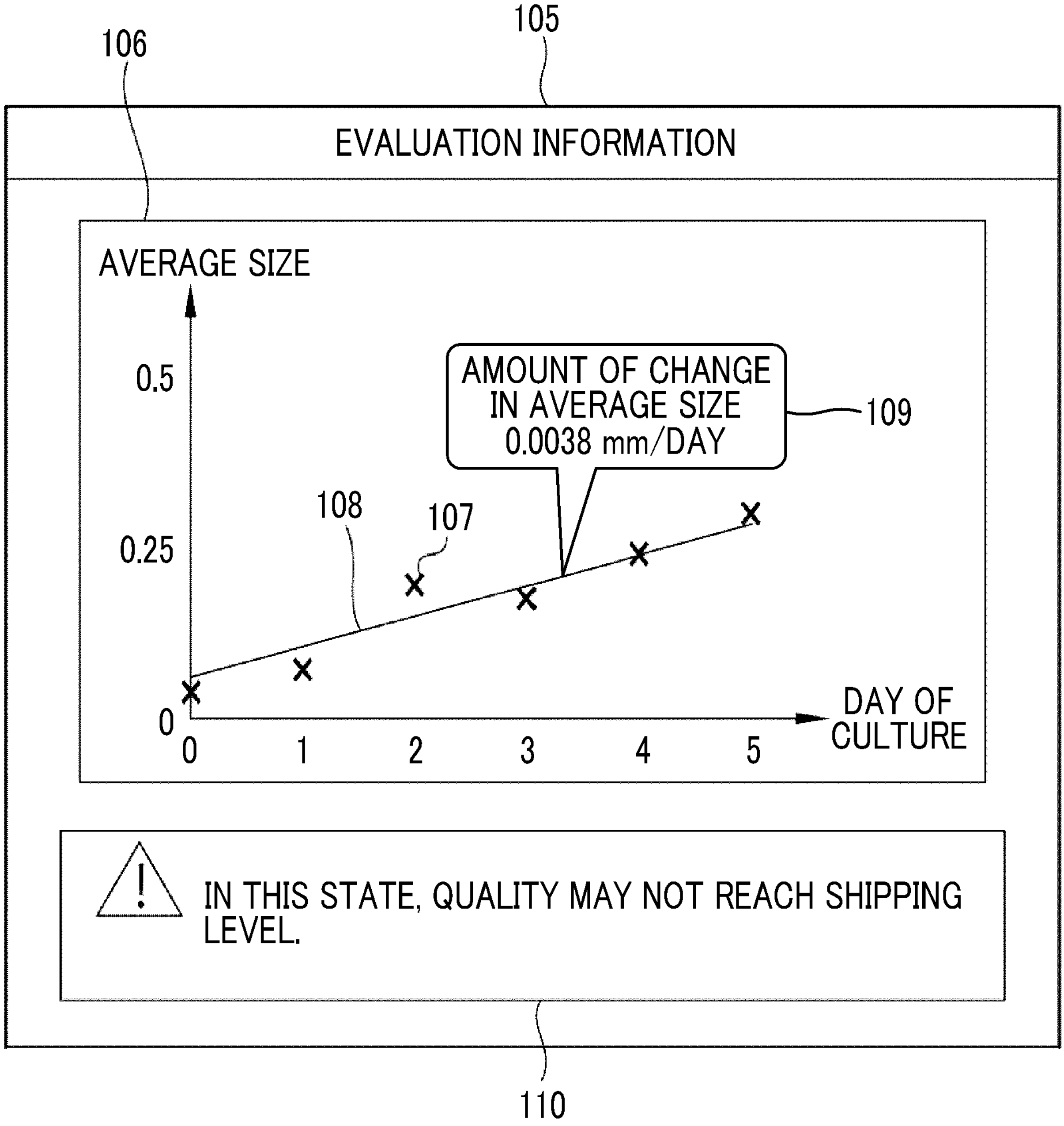
105
106
EVALUATION INFORMATION
AVERAGE SIZE
0.5
0.25
0
AMOUNT OF CHANGE
IN AVERAGE SIZE
0.0038 mm/DAY
109
108
107
DAY OF
CULTURE
0
1
2
3
4
5
IN THIS STATE, QUALITY MAY NOT REACH SHIPPING LEVEL.
110

FIG. 22

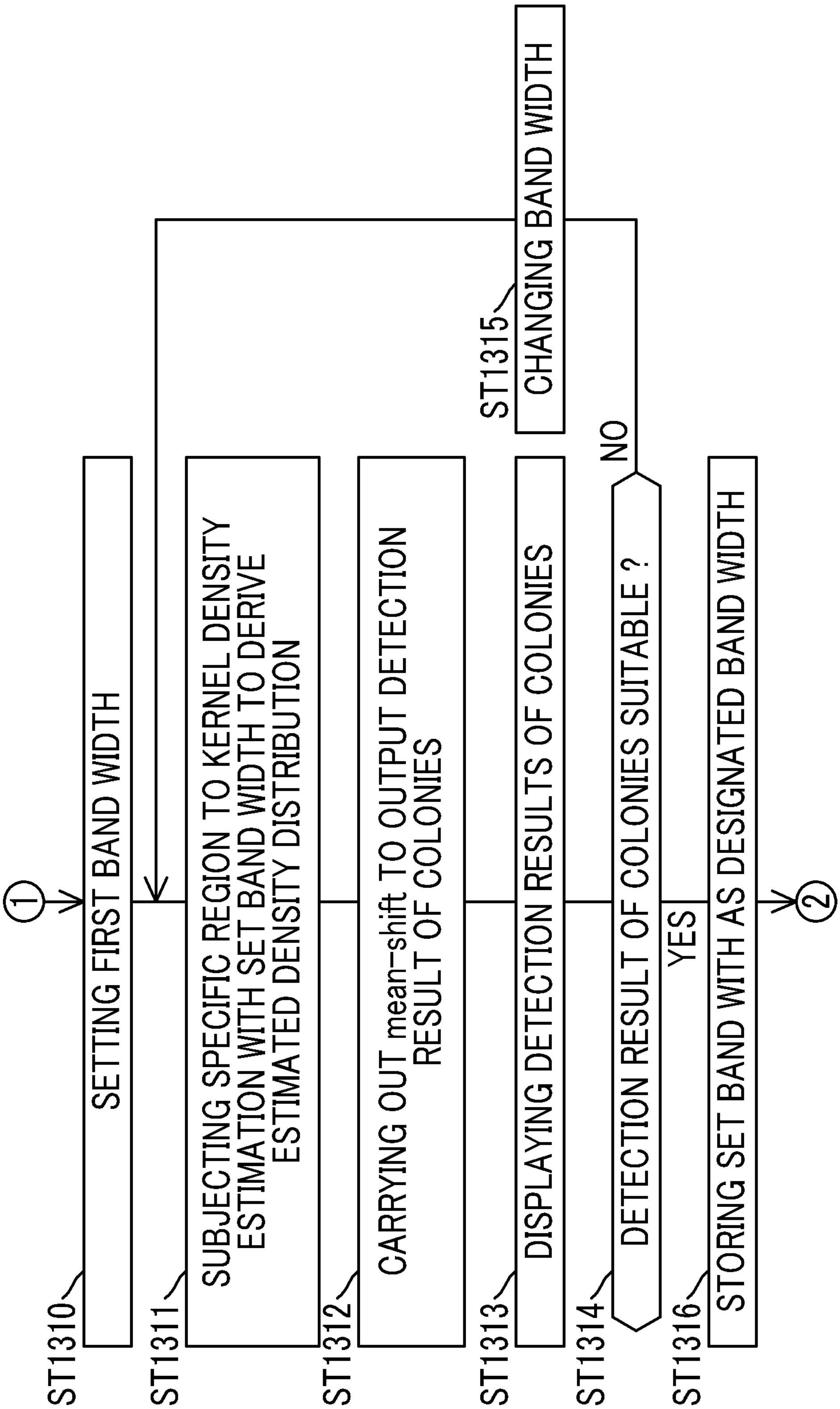
1
ST1310
SETTING FIRST BAND WIDTH
ST1311
SUBJECTING SPECIFIC REGION TO KERNEL DENSITY ESTIMATION WITH SET BAND WIDTH TO DERIVE ESTIMATED DENSITY DISTRIBUTION
ST1312
CARRYING OUT mean-shift TO OUTPUT DETECTION RESULT OF COLONIES
ST1313
DISPLAYING DETECTION RESULTS OF COLONIES
ST1314
DETECTION RESULT OF COLONIES SUITABLE ?
NO
ST1315
CHANGING BAND WIDTH
YES
ST1316
STORING SET BAND WITH AS DESIGNATED BAND WIDTH
2

FIG. 23

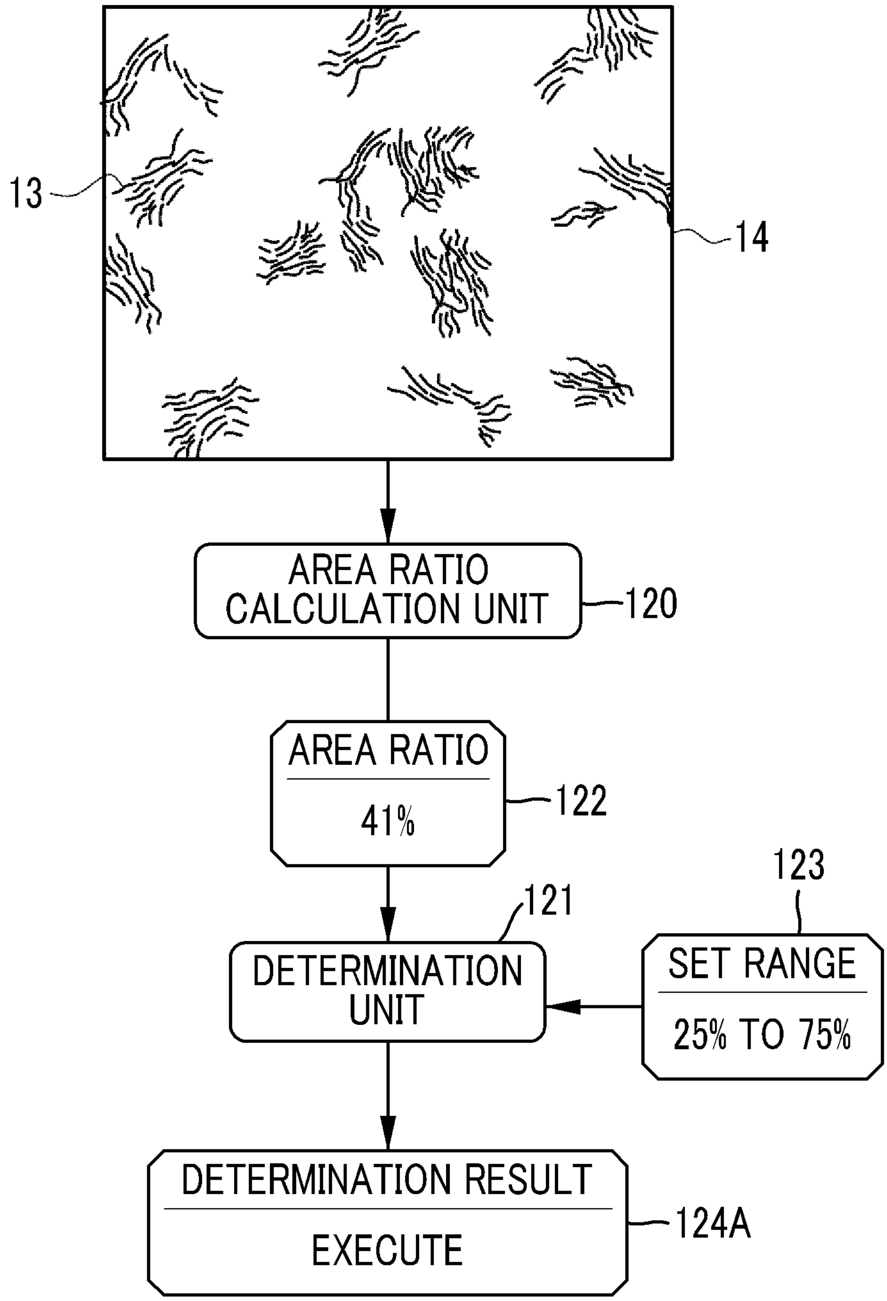
13
14
AREA RATIO
CALCULATION UNIT
120
AREA RATIO
41%
122
123
121
DETERMINATION
UNIT
SET RANGE
25% TO 75%
DETERMINATION RESULT
EXECUTE
124A

FIG. 24

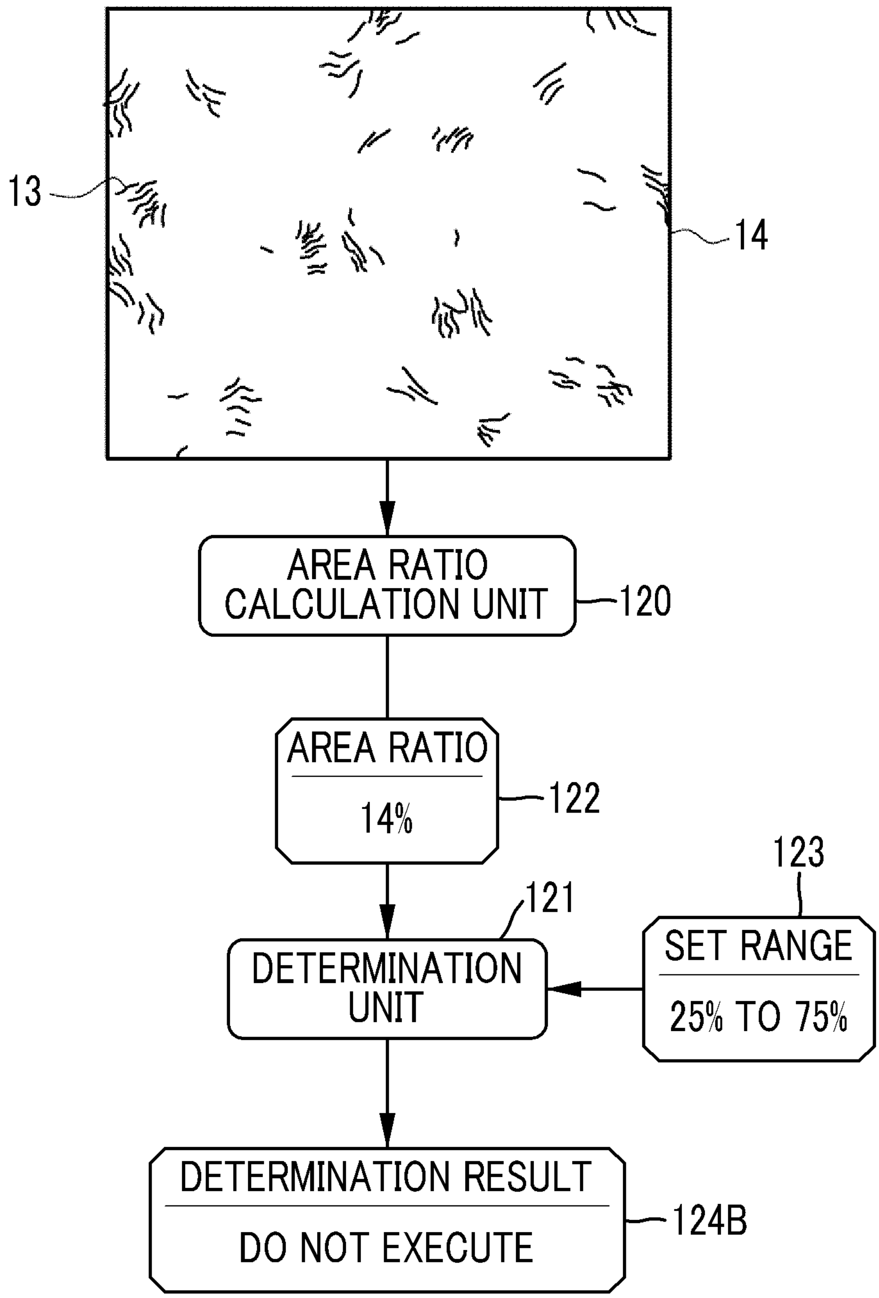
13
14
AREA RATIO
CALCULATION UNIT
120
AREA RATIO
14%
122
123
121
DETERMINATION
UNIT
SET RANGE
25% TO 75%
DETERMINATION RESULT
DO NOT EXECUTE
124B

FIG. 25

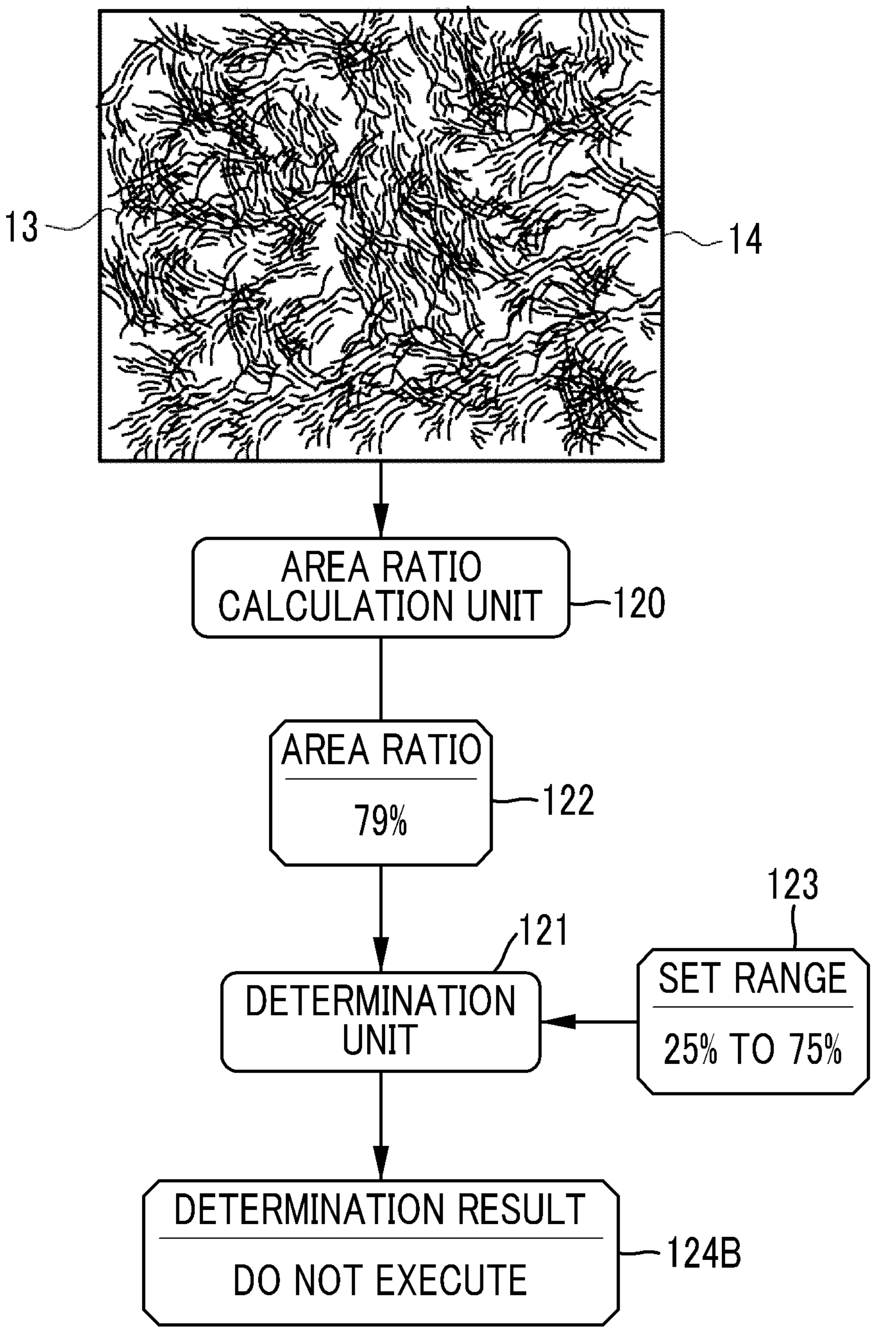
13
14
AREA RATIO CALCULATION UNIT
120
AREA RATIO
79%
122
123
121
DETERMINATION UNIT
SET RANGE
25% TO 75%
DETERMINATION RESULT
DO NOT EXECUTE
124B

FIG. 26

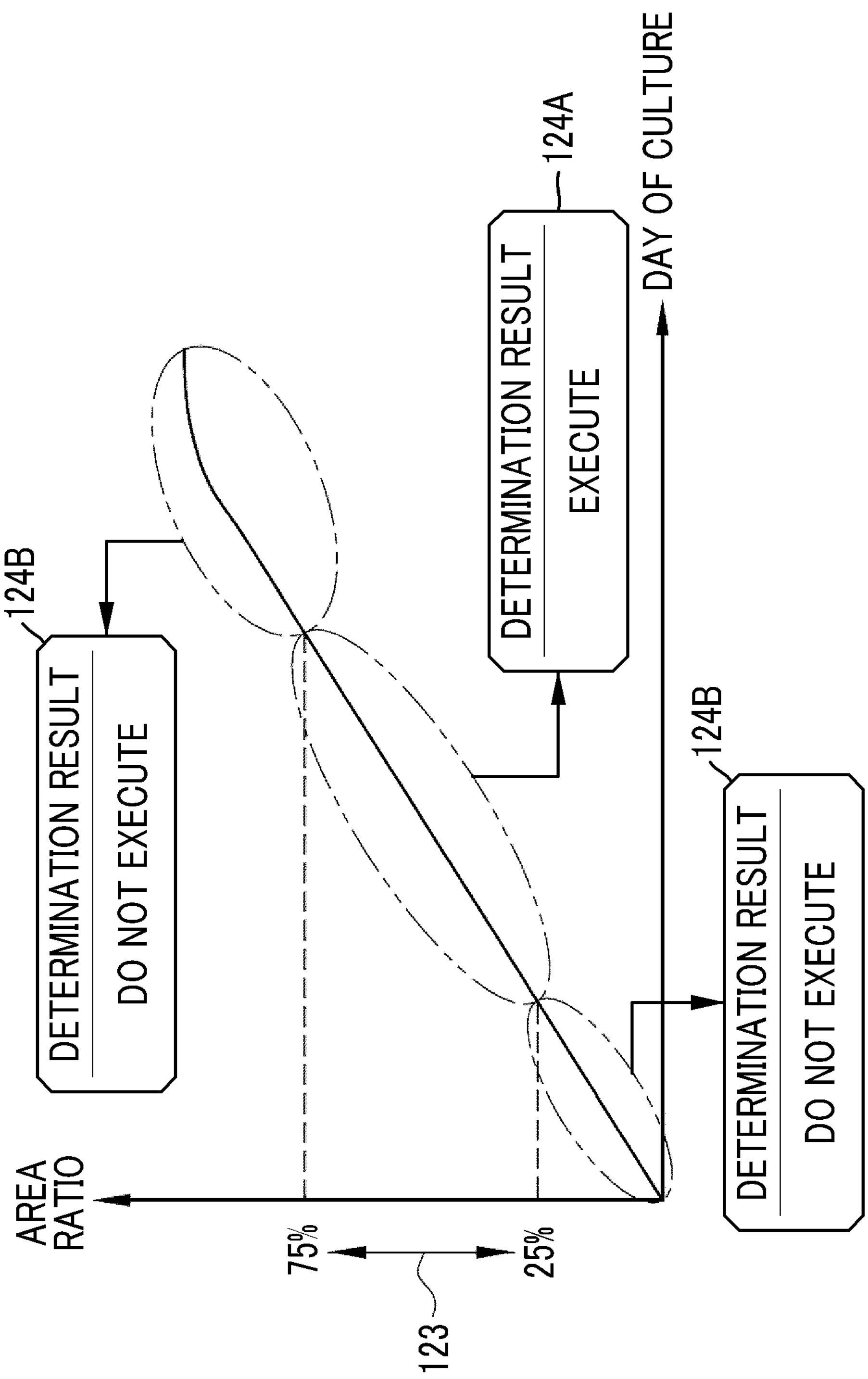
AREA RATIO
DAY OF CULTURE
75%
25%
123
124A
124B
124B
DETERMINATION RESULT
DO NOT EXECUTE
DETERMINATION RESULT
EXECUTE
DETERMINATION RESULT
DO NOT EXECUTE

FIG. 27

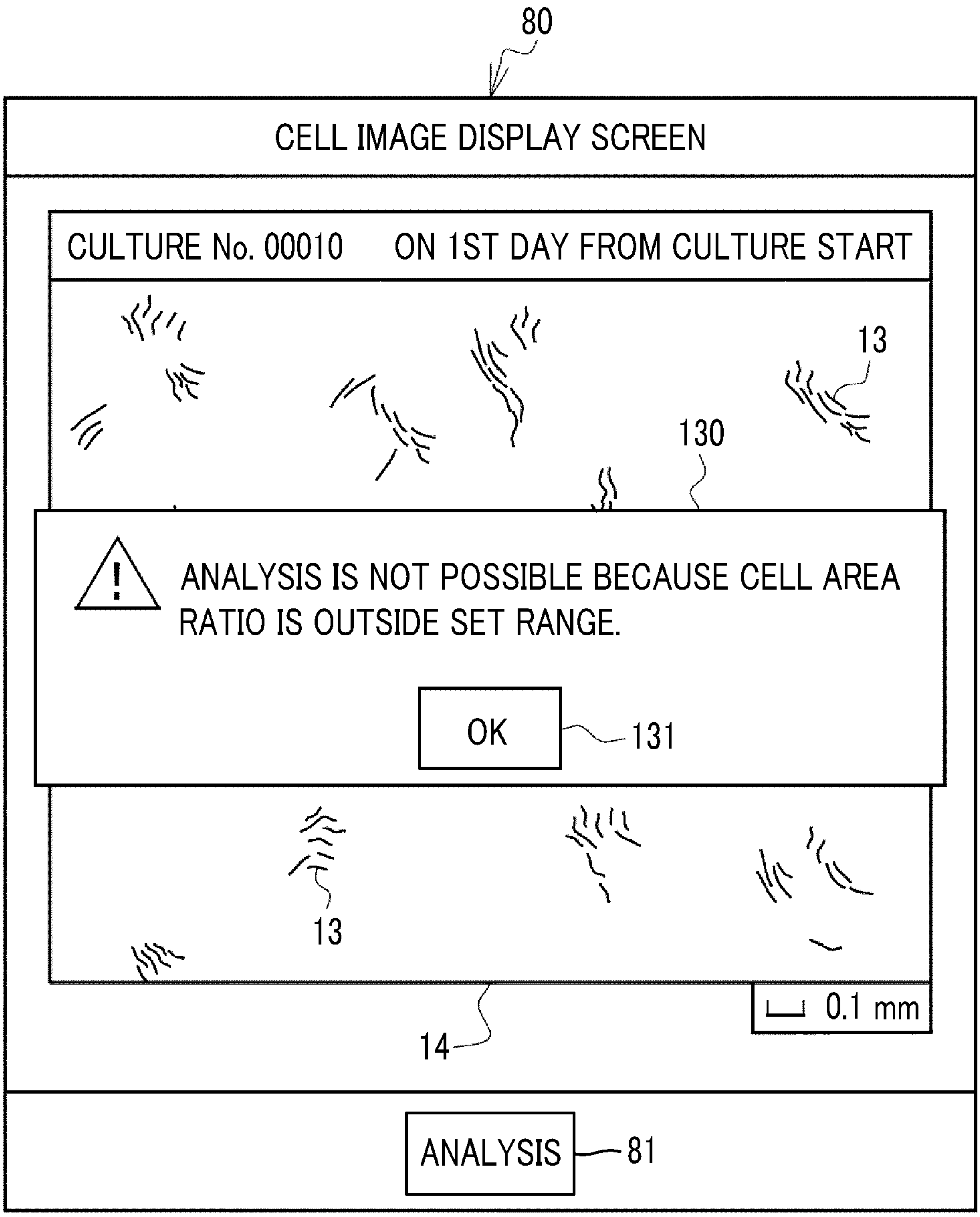
80
CELL IMAGE DISPLAY SCREEN
CULTURE No. 00010
ON 1ST DAY FROM CULTURE START
13
130
ANALYSIS IS NOT POSSIBLE BECAUSE CELL AREA RATIO IS OUTSIDE SET RANGE.
OK
131
13
0.1 mm
14
ANALYSIS
81

FIG. 28

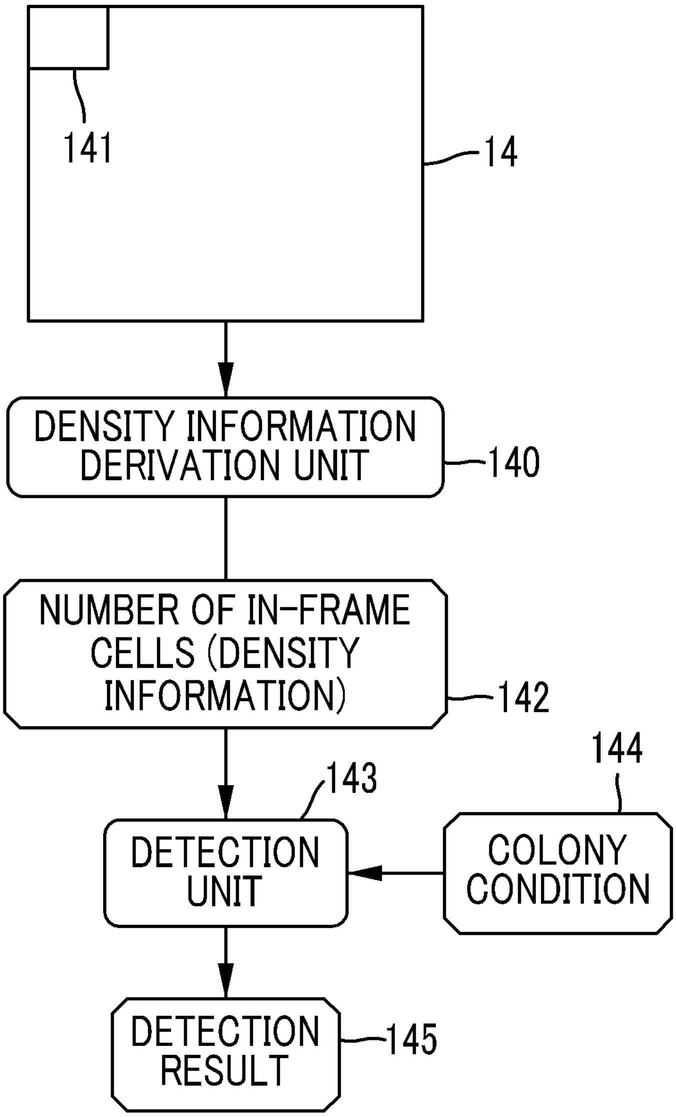
141
14
DENSITY INFORMATION DERIVATION UNIT
140
NUMBER OF IN-FRAME CELLS (DENSITY INFORMATION)
142
143
144
DETECTION UNIT
COLONY CONDITION
DETECTION RESULT
145

FIG. 29

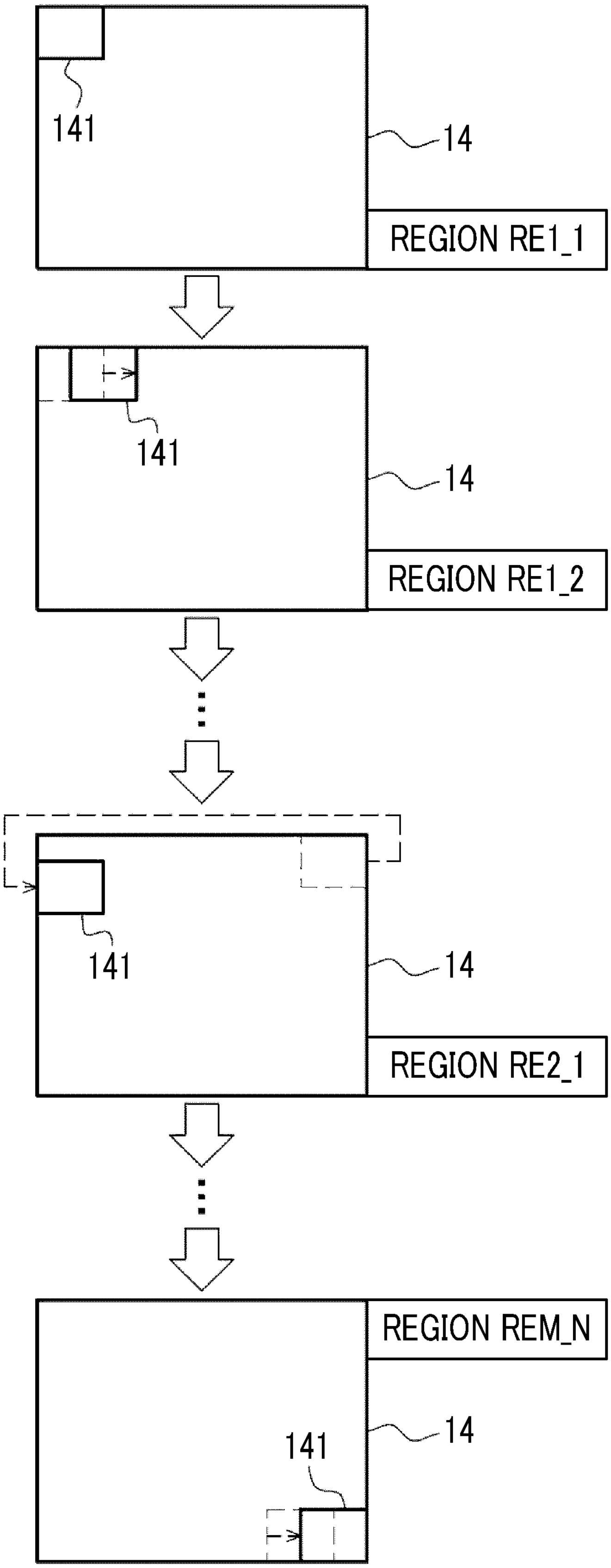
141
14
REGION RE1_1
141
14
REGION RE1_2
141
14
REGION RE2_1
REGION REM_N
14
141

FIG. 30

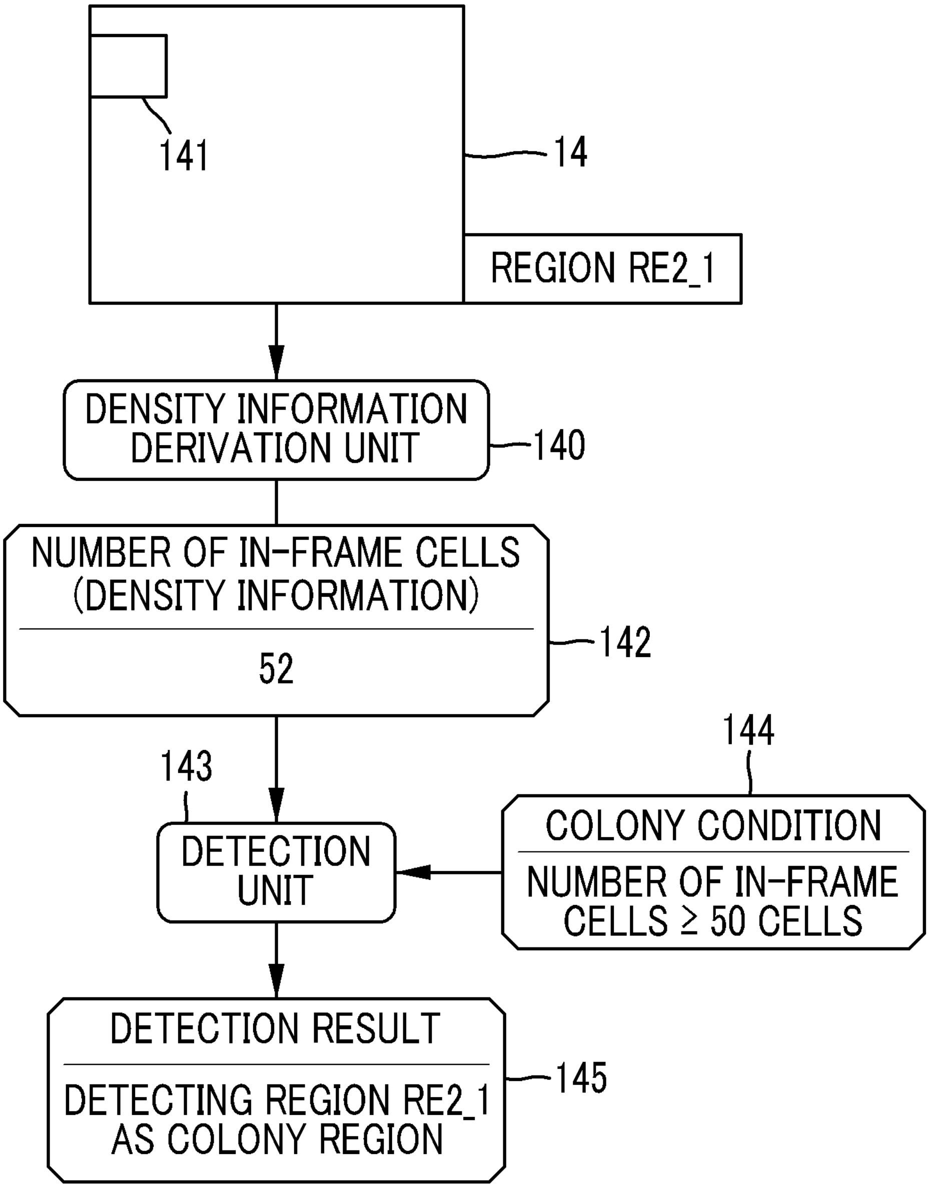
141
14
REGION RE2_1
DENSITY INFORMATION DERIVATION UNIT
140
NUMBER OF IN-FRAME CELLS (DENSITY INFORMATION)
52
142
143
144
DETECTION UNIT
COLONY CONDITION
NUMBER OF IN-FRAME CELLS ≥ 50 CELLS
DETECTION RESULT
DETECTING REGION RE2_1 AS COLONY REGION
145

FIG. 31

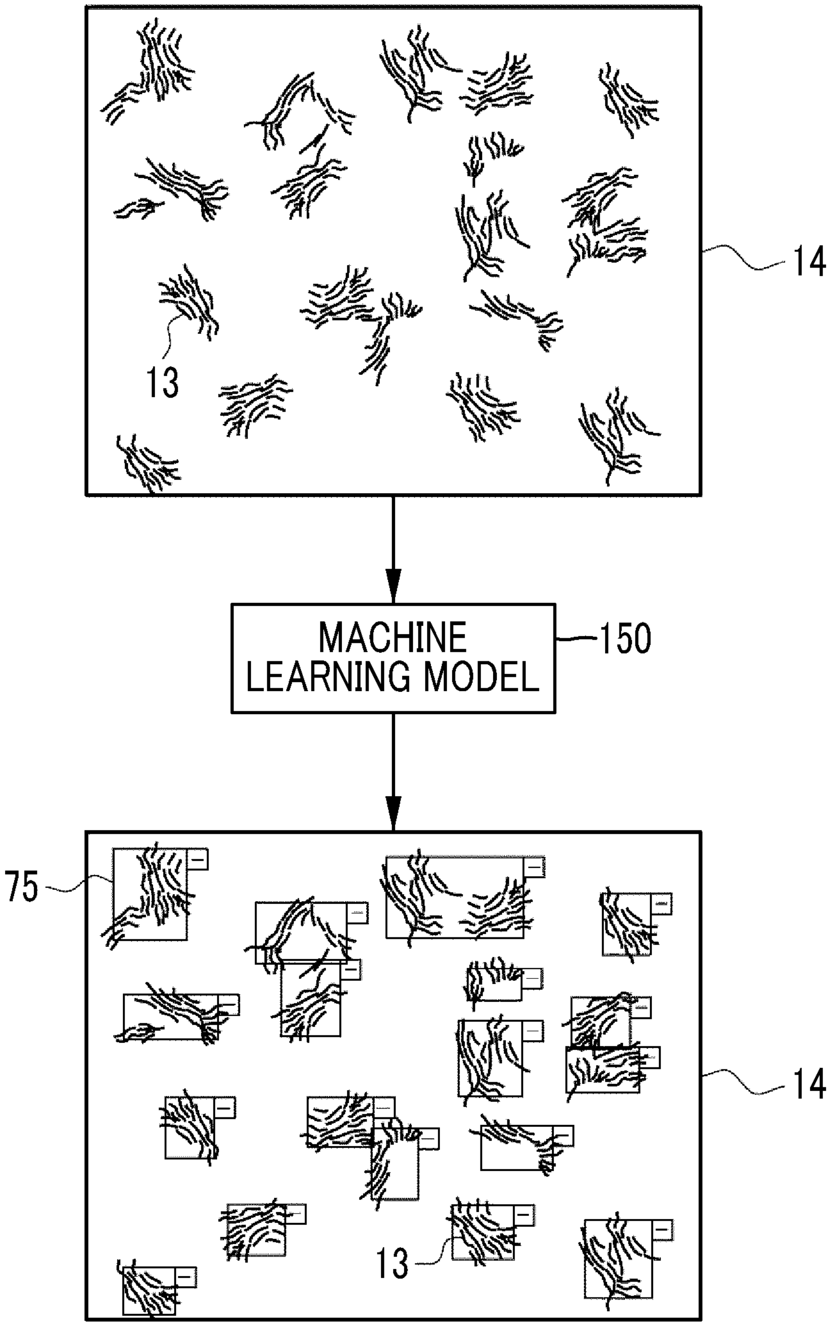

FIG. 32

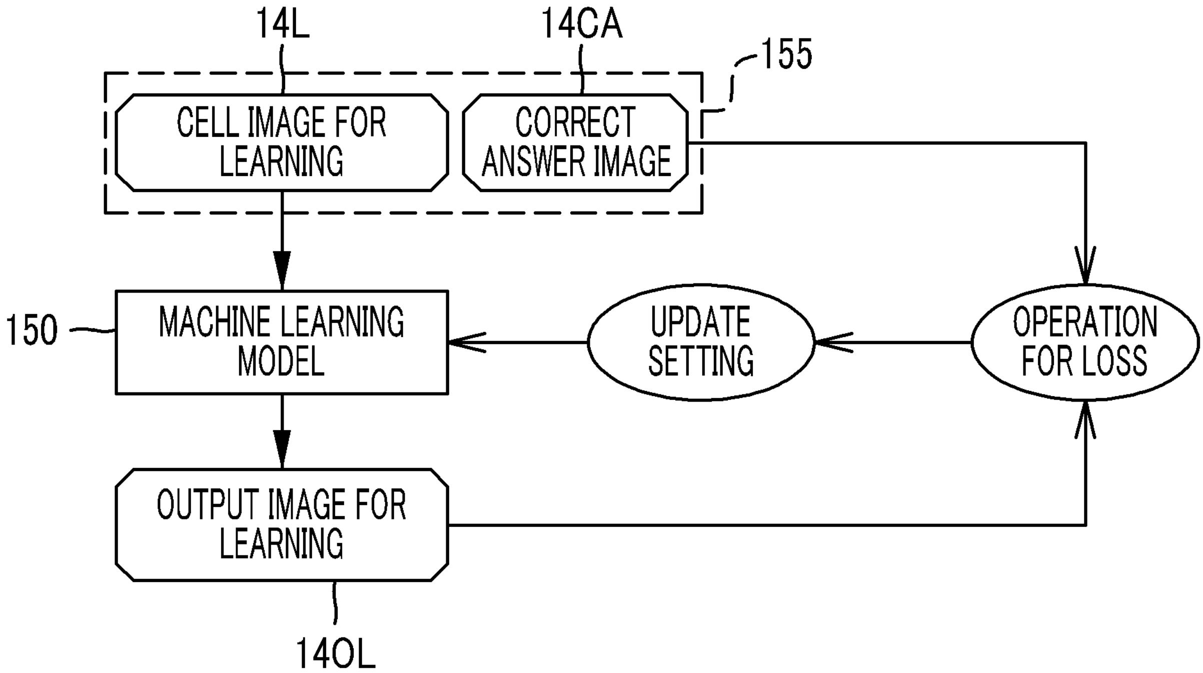
14L
14CA
155
CELL IMAGE FOR LEARNING
CORRECT ANSWER IMAGE
150
MACHINE LEARNING MODEL
UPDATE SETTING
OPERATION FOR LOSS
OUTPUT IMAGE FOR LEARNING
14OL

## INTERNATIONAL SEARCH REPORT

International application No.
**PCT/JP2021/047431**

**A. CLASSIFICATION OF SUBJECT MATTER**

***A61K 35/28***(2015.01)i; ***A61K 35/32***(2015.01)i; ***A61P 19/02***(2006.01)i; ***C12Q 1/06***(2006.01)i; ***G01N 33/48***(2006.01)i
FI: A61K35/28; A61K35/32; A61P19/02; C12Q1/06; G01N33/48 M

According to International Patent Classification (IPC) or to both national classification and IPC

**B. FIELDS SEARCHED**

Minimum documentation searched (classification system followed by classification symbols)

A61K35/28; A61K35/32; A61P19/02; C12Q1/06; G01N33/48

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSISI (STN)

**C. DOCUMENTS CONSIDERED TO BE RELEVANT**

| Category* | Citation of document, with indication, where appropriate, of the relevant passages | Relevant to claim No. |
|---|---|---|
| X<br><br><br>A | JP 2010-501547 A (TOKYO MEDICAL AND DENTAL UNIVERSITY) 21 January 2010 (2010-01-21)<br>claims, paragraphs [0024], [0027], [0028], examples, fig. 2 | 11<br><br><br>1-10 |
| X<br><br><br><br>A | SEKIYA, I. et al. Additional Use of Synovial Mesenchymal Stem Cell Transplantation Following Surgical Repair of a Complex Degenerative Tear of the Medial Meniscus of the Knee: A Case Report, Cell Transplant., 2019, vol. 28, issue 11, pp. 1445-1454<br>in particular, abstract, procedures, results | 11<br><br><br><br>1-10 |
| A | JP 2019-004794 A (DAINIPPON PRINTING CO., LTD.) 17 January 2019 (2019-01-17)<br>claims, paragraphs [0002], [0013]-[0051] | 1-10 |
| A | JP 2016-158577 A (FUJIFILM CORP.) 05 September 2016 (2016-09-05)<br>paragraphs [0069]-[0101], fig. 11 | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 February 2022** | **01 March 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br><br>Telephone No. |

Form PCT/ISA/210 (second sheet) (January 2015)

**INTERNATIONAL SEARCH REPORT**

International application No.
**PCT/JP2021/047431**

**C. DOCUMENTS CONSIDERED TO BE RELEVANT**

| Category* | Citation of document, with indication, where appropriate, of the relevant passages | Relevant to claim No. |
|---|---|---|
| A | JP 2014-531212 A (OBSHHESTVO S OGRANICHENNOI OTVETSTVENNOST'YU "VITACEL") 27 November 2014 (2014-11-27)<br>claims, paragraphs [0037]-[0043] | 1-10 |
| P, X | WO 2021/240986 A1 (FUJIFILM CORP.) 02 December 2021 (2021-12-02)<br>claims, paragraphs [0002], [0019]-[0085] | 1-11 |
| P, X | JP 6864302 B1 (FUJIFILM CORP.) 28 April 2021 (2021-04-28)<br>claims, examples | 11 |
| P, A | | 1-10 |
| P, X | JP 6864303 B1 (FUJIFILM CORP.) 28 April 2021 (2021-04-28)<br>claims, examples | 11 |
| P, A | | 1-10 |

Form PCT/ISA/210 (second sheet) (January 2015)

**INTERNATIONAL SEARCH REPORT**
**Information on patent family members**

International application No.
**PCT/JP2021/047431**

| Patent document cited in search report | Publication date (day/month/year) | Patent family member(s) | Publication date (day/month/year) |
|---|---|---|---|
| JP 2010-501547 A | 21 January 2010 | WO 2008/023829 A1<br>claims, paragraphs [0039], [0042], [0043], examples, fig. 2<br>US 2010/0178274 A1 | |
| JP 2019-004794 A | 17 January 2019 | (Family: none) | |
| JP 2016-158577 A | 05 September 2016 | (Family: none) | |
| JP 2014-531212 A | 27 November 2014 | WO 2013/051963 A1<br>claims, pp. 9-11<br>US 2013/0266550 A1<br>EP 2764091 A1 | |
| WO 2021/240986 A1 | 02 December 2021 | (Family: none) | |
| JP 6864302 B1 | 28 April 2021 | (Family: none) | |
| JP 6864303 B1 | 28 April 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019004794 A
- JP 2021040648 A


### Non-patent literature cited in the description


- **PROCKOP, D.J.** *Science,* 1997, vol. 276, 71-4
- **ZUK, P.A. et al.** *Mol Biol Cell,* 2002, vol. 13, 4279-95
- **CAO et al.** *Nat Cell Biol,* 2003, vol. 5, 640-6
- **DE BARI, C. et al.** *Arthritis Rheum,* 2001, vol. 44, 1928-42
- **FUKUMOTO, T. et al.** *Osteoarthritis Cartilage,* 2003, vol. 11
- **SAKAGUCHI et al.** *Arthritis Rhum,* 2005, vol. 52, 2521-9